# EUROPEAN PATENT APPLICATION

(11) **EP 0 799 834 A1**
(43) Date of publication of application: **08.10.1997**
(21) Application number: 96810216.0
(22) Date of filing: 04.04.1996
(51) Int. Cl.: C07H 19/04, A61K 31/70

(54) **Modified nucleotides**

(71) Applicant: Novartis AG, 4058 Basel (CH)
(72) Inventor: Marx, Andreas, 79639 Grenzach-Wyhlen, (DE); Giese, Bernd, Prof.Dr., 4051 Basel (CH)

(57) **Abstract**

The current invention concerns new modified nucleotides that are accepted by reverse transcriptases and incorporated in to a growing oligonucleotide but are not accepted by polymerases. Oligonucleotides comprising the new modified nucleotides can be cleaved photolytically. Also embraced is a process for the synthesis of the new modified nucleotides, and their use.

Sequence listing

(1) GENERAL INFORMATION:

(i) APPLICANT:
(A) NAME: CIBA-GEIGY AG
(B) STREET: Klybeckstr. 141
(C) CITY: Basel
(E) COUNTRY: Switzerland
(F) POSTAL CODE (ZIP): 4002
(G) TELEPHONE: +41 61 69 11 11
(H) TELEFAX: + 41 61 696 79 76
(I) TELEX: 962 991

(ii) TITLE OF INVENTION: Modified Nucleotides

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 1:

GTGGTGCGAA TTCTGTGGAT 20

(2) INFORMATION FOR SEQ ID NO: 2:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:complement (1..40)

(xi) SEQUENCE DESCRIPTION: SEQ ID NO: 2:

CACCACGCTT AAGACACCTA GTCGTTCCTA CTTGCTGGCT 40

## Description

Antibiotics from the enediyne family like neocarzinostatin and esperamycin as well as metal complexes of bleomycin glycopeptides induce the oxidative cleavage of DNA by generation of highly reactive DNA radicals. In a similar way, chemical nucleases and drugs can generate oxidative stress by damage of DNA *via* radicals. The research in this area is focused on the development of new selective DNA cleavers and on the elucidation of the mechanism of action of these compounds. Whereas hydroxyl radicals react rather unselectively, several agents whose active center are bound to the minor groove preferentially abstract H-atoms from the 4 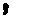 -and/or 5 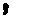 -position of the deoxyribose. Recently it has been demonstrated that 4 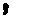 -deoxyribosyl radicals can be generated selectively from selenides (Giese *et al., Synthesis* (1994) 1310-1312) or ketones (Giese *et al., Synlett* (1994), 1003) by photolysis. These artificial oligonucleotides could be synthesized by solid-phase synthesis with suitable substituted mononucleotides. Now, different synthetic strategies towards 4 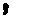 -acyl-2 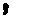 -deoxy-nucleotides with improved properties have been worked out.

Another important feature of nucleotide analogs is their use as antiviral compounds, as for example in the treatment of AIDS or some sarcomas. Currently used nucleotide analogues like 3 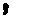 -azido-2 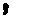 ,3 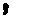 -didesoxythymidine (AZT); 2 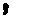 ,3 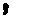 -didesoxy-2 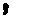 ,3 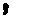 -didehydrothymidine (d4T); 2 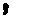 ,3 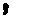 -didesoxyinosine (ddI) or 2 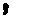 ,3 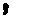 -didesoxycytidine (ddC) that are accepted by reverse transcriptases are also accepted, to a lower extent, by eukaryotic polymerases. Accordingly, when used in higher concentrations over a prolonged period the limited selectivity of these nucleotide analogues cause severe side effects.

Surprisingly, new modified nucleotides have been found that are accepted by reverse transcriptases and incorporated in to a growing oligonucleotide but not by polymerases. Usually the synthesis stops after incorporation of the inventive nucleotides. However, under certain conditions, the reverse transcriptase can be forced to continue chain elongation after incorporation of the inventive nucleotide to create an oligonucleotide that can be cleaved by light irradiation.

### Detailed description of the invention

The current invention concerns a compound of formula 1 or 2 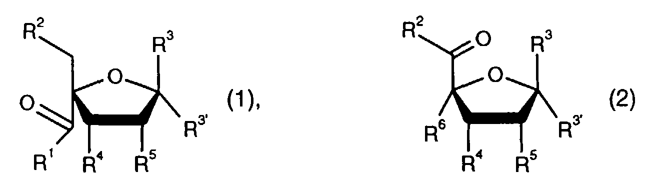 wherein
R¹ is CH₃, C₂H₅, CH₂X, CHX₂, CX₃, C(O)H, C(O)C₁-C₄alkyl, CH₂OH, CH₂-O-C₁-C₄alkyl, CH₂-O-phenyl, phenyl, or phenyl substituted with nitro or X; preferred are CH₃, C₂H₅, CF₃, CH₃OH, CH₃-O-CH₃, phenyl and phenyl substituted with nitro; more preferred are CH₃; C₂H₅ and phenyl; most preferred is CH₃;
R² is OR⁷, mono-, di-, or triphosphate, or esters or prodrugs thereof; preferred are HO and mono-, di-, or triphosphate;
one of the residues R³ and R³ 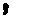 is a purine or pyrimidine residue or an analogue thereof, and the other is hydrogen; preferred purine or pyrimidine radicals or an analogues thereof are adenine, inosine, N-methyladenine, N-benzoyladenine, 2-methylthioadenine, 2-aminoadenine, 6-hydroxypurine, 2-amino-6-chloropurine, 2-amino-6-methylthiopurine, guanine, N-isobutyrylguanine, uracil, thymine, cytosine, 5-fluorouracil, 5-chlorouracil, 5-bromouracil, dihydrouracil and 5-methylcytosine; and their base-protected derivatives; more preferred are adenine, inosine, guanine, uracil, thymine or cytosine; and most preferred is thymine; in a further preferred embodiment R³ 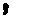 is hydrogen;
R⁴ and R⁵ are independent of one another H, OR⁷, O-C₁-C₄alkylNHR⁷, O-C₁-C₄alkylNR⁷₂,-O-(CH₂-CH₂-O)₁₋₄R⁷ or -O-CH₂-C(OR⁷)H-CH₂-OR⁷; preferred are H, and OR⁷; in a more preferred embodiment R⁴ is OH and R⁵ is H;
R⁶ is H, OH, CH₂OH, CH₃, CH₂CH₃, CH₂CH₂OH; and preferably H, OH or CH₂OH;
R⁷ is H or an NH or OH-protecting group; preferred is H or C₁-C₄alkyl; more preferred is H;
X is F, Cl, Br or I; preferably F or Cl; and more preferably F;
or a pharmaceutically acceptable salt or a prodrug thereof.

In a preferred embodiment of the invention the inventive compound is of formula 1.

Purine or pyrimidine residues or analogues thereof are well known in the art as for example in WO-A-9520597 and Périgaud *et al*, Nucleosides and Nucleotides (1992), **11**, 903-945.

Protective groups and processes for derivatisation of hydroxyl groups with such protective groups are generally known in sugar and nucleotide chemistry and described, for example, by B. T. Greene, Protective Groups in Organic Synthesis, Wiley Interscience, New York (1991). Examples of such protective groups are: linear or branched C₁-C₈alkyl, particularly C₁-C₄alkyl, for example methyl, ethyl, n- and i-propyl, n-, i- and t-butyl; C₇-C₁₈aralkyl, for example benzyl, methylbenzyl, dimethylbenzyl, methoxybenzyl, dimethoxybenzyl, bromobenzyl, diphenylmethyl, di(methylphenyl)methyl, di(dimethylphenyl)methyl, di(methoxyphenyl)methyl, di(dimethoxyphenyl)methyl, trityl, tri(methylphenyl)methyl, tri(dimethylphenyl)methyl, methoxyphenyl(diphenyl)methyl, di(methoxyphenyl)phenylmethyl, tri(dimethoxyphenyl)methyl, tri(methoxyphenyl)methyl; triphenylsilyl, alkyldiphenylsilyl, dialkylphenylsilyl and trialkylsilyl having 1 to 20, preferably 1 to 12 and particularly preferably 1 to 8, C atoms in the alkyl groups, for example trimethylsilyl, triethylsilyl, tri-n-propylsilyl, i-propyldimethylsilyl, t-butyldimethylsilyl, t-butyldiphenylsilyl, n-octyldimethylsilyl, (1,1,2,2-tetramethylethyl)dimethylsilyl; -(C₁-C₈alkyl)₂Si-O-Si(C₁-C₈alkyl)₂-, in which alkyl is, e.g., methyl, ethyl, n- or i-propyl, n-, i- or t-butyl; C₂-C₁₂acyl, particularly C₂-C₈acyl, for example acetyl, propanoyl, butanoyl, pentanoyl, hexanoyl, benzoyl, methoxybenzoyl, methylbenzoyl, chlorobenzoyl and bromobenzoyl; R¹²-SO₂-, in which R¹² is C₁-C₁₂alkyl, particularly C₁-C₆alkyl, C₅- or C₆cycloalkyl, phenyl, benzyl, C₁-C₁₂alkylphenyl and particularly C₁-C₄alkylphenyl, or C₁-C₁₂alkylbenzyl and particularly C₁-C₄alkylbenzyl, or halophenyl or halobenzyl, for example methyl-, ethyl-, propyl-, butyl-, phenyl-, benzyl-, p-bromo-, p-methoxy- or p-methylphenylsulfonyl; unsubstituted or F-, Cl-, Br-, C₁-C₄alkoxy-, tri(C₁-C₄alkyl)silyl- or C₁-C₄alkylsulfonyl-substituted C₁-C₁₂alkoxycarbonyl, preferably C₁-C₈alkoxycarbonyl, for example methoxy-, ethoxy-, n- or -propoxy- or n-, i- or t-butoxycarbonyl, 2-trimethylsilylethoxycarbonyl, 2-methylsulfonylethoxycarbonyl, or phenoxycarbonyl or benzyloxycarbonyl which is unsubstituted or substituted as for alkoxycarbonyl, for example methyl-or methoxy-or chlorophenoxycarbonyl or -benzyloxycarbonyl, and also 9-fluorenylmethyloxycarbonyl.

If the protecting group is alkyl, it can be substituted by F, Cl, Br, C₁-C₄alkoxy, phenoxy, chlorophenoxy, methoxyphenoxy, benzyloxy, methoxybenzyloxy or chlorophenoxy.

In a preferred embodiment, the protective groups are, independently of one another, linear or branched C₁-C₄alkyl, C₇-C₁₈aralkyl, trialkylsilyl having 1 to 12 C atoms in the alkyl groups; -(C₁-C₄alkyl)₂Si-O-Si(C₁-C₄alkyl)₂ like (CH₃)₂Si-O-Si(CH₃)₂- and -(i-C₃H₇)₂Si-O-Si(iC₃H₇)₂-; C₂-C₈acyl, R¹²-SO₂-, in which
R¹² is C₁-C₆alkyl; phenyl or benzyl unsubstituted or substituted with F, Cl or Br; C₁-C₄alkylphenyl; C₁-C₄alkylbenzyl; C₁-C₈alkoxycarbonyl; phenoxycarbonyl; benzyloxycarbonyl or 9-fluorenylmethoxycarbonyl.

In a particularly preferred embodiment, the protective groups are methyl, ethyl, n-or i-propyl, n-, i- or t-butyl; benzyl, methylbenzyl, dimethylbenzyl, methoxybenzyl, dimethoxybenzyl, bromobenzyl; diphenylmethyl, di(methylphenyl)methyl, di(dimethylphenyl)methyl, di(methoxyphenyl)methyl, di(methoxyphenyl)(phenyl)methyl, trityl, tri(methylphenyl)methyl, tri(dimethylphenyl)methyl, tri(methoxyphenyl)methyl, tri(dimethoxyphenyl)methyl; trimethylsilyl, triethylsilyl, tri-n-propylsilyl, i-propyldimethylsilyl, t-butyldimethylsilyl, t-butyldiphenylsilyl, n-octyldimethylsilyl, (1,1,2,2-tetramethylethyl)dimethylsilyl, -(i-C₃H₇)₂Si-O-Si(i-C₃H₇)₂-,-(CH₃)₂-Si-O-Si(CH₃)₂-; C₁-C₈acyl groups like acetyl, propanoyl, butanoyl, pentanoyl, hexanoyl, benzoyl, methylbenzoyl, methoxybenzoyl, chlorobenzoyl and bromobenzoyl; methyl-, ethyl-, propyl-, butyl-, phenyl-, benzyl-, p-bromo-, p-methoxy- and p-methylphenylsulfonyl; methoxy-, ethoxy-, n- or i-propoxy- or n-, i- or t-butoxycarbonyl, or phenoxycarbonyl, benzyloxycarbonyl, methyl- or methoxy- or chlorophenoxycarbonyl or -benzyloxycarbonyl or 9-fluorenylmethoxycarbonyl.

Even more preferred protective groups are C₁-C₈acyl groups, for example acetyl, propanoyl, butanoyl and benzoyl.

To increase the bioavailability, the inventive compounds may be used also in form of pro-drugs, that are converted within the organism to an active agent. Usually, suitable prodrugs are modified mono- and diphosphates. These prodrugs are altered *in vivo* (by the cells) to mono- or diphosphates and than converted to triphosphates. Suitable prodrugs are described, for example, in Jones & Bischofsberger, Antiviral Res. (1995), 27, 1-17; Meier, Angew. Chem. (1996), **108**, 77-78; Nillroth *et al*., Antiviral Res. (1995), **6**, 50-64; Shimizu *et al*., Nucleosides and nucleotides (1992) **11**, 583-594; Hostetler *et al*, Proc. Natl. Acad. Sci. USA (1993), **90**, 11835-11839. Some examples are phosphonomethyl ether, S-acyl-2-thioethyl esters (SATE), dithioethyl esters, acyloxybenzyl derivates, phosphoramidates, triaryl esters, activated alkylesters, glucosyl triester, 2-nucleos-5 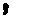 -O-yl-4H-1,3,2-benzodioxaphosphinin-2-oxids, dimyristoylglycerol and the like.

Also embraced by the scope of the invention are pharmaceutically acceptable salts with conventional therapeutically acceptable acids or bases. Representative inorganic acids are hydrohalic acids (such as hydrochloric acid), and also sulfuric acid, phosphoric acid and pyrophosphoric acid. Representative organic acids are in particular arene-sulfonic acids (such as benzenesulfonic or p-toluenesulfonic acid), or lower alkanesulfonic acids (such as methanesulfonic acid), as well as carboxylic acids such as acetic acid, lactic acid, palmitic acid, stearic acid, malic acid, tartaric acid, ascorbic acid and citric acid. Representative organic bases are, e.g. sodium, potassium, calcium or magnesium salts, or also ammonium salts derived from ammonia or a pharmacologically acceptable organic nitrogen-containing base. However, if the inventive compound contains at the same time free carboxyl groups and free amino groups, it can also be obtained in the form of an inner salt.

A pharmacologically acceptable salt can be obtained from the free compound by reaction with acids, e.g. with those acids which form the above-mentioned salts, and by evaporation or lyophilization, or by adjusting the pH to a suitable neutral point, and by evaporation or lyophilization.

Another embodiment of the invention concerns the use of the inventive compound in a method of treatment. For example, one possible application is the integration of the modified nucleotides *via* the increased activity of tumor cells into the newly synthesized DNA and to kill the cells afterwards by irradiation with light at a certain wave length (by cleaving the newly synthesized DNA into pieces). Another possibility is the treatment of retro virus induced diseases like AIDS and some cancer. In a preferred embodiment of the invention the inventive compounds are used in a method of treating AIDS and retrovirus dependent cancer like, virus induced leukemia, lymphoma and sarcoma. For this purpose, the claimed active inventive compound may be administered directly or it is formed *in vivo* from a prodrug, as already explained above.

Another embodiment of the invention concerns the use of the inventive compound in a method of inhibiting the proliferation of retro viruses. Typical retrovirus are, for example, MMTV, MLV, spumavirus, HTLV, BLV, lentivirus, HIV, SIV and HSV.

The invention likewise relates to a pharmaceutical composition comprising a compound according to the invention or pharmaceutically acceptable salts thereof as active ingredients, optionally together with pharmaceutically acceptable carrier and to processes for their preparation.

The pharmaceutical preparations according to the invention which contain the compound according to the invention or pharmaceutically acceptable salts thereof are those for enteral, such as oral, furthermore rectal, and parenteral administration to a warm-blooded animal, the pharmacological active ingredient being present on its own or together with a pharmaceutically acceptable carrier. The daily dose of the active ingredient depends on the age and the individual condition and also on the manner of administration.

The pharmaceutical preparations according to the invention which contain the compound according to the invention or pharmaceutically acceptable salts thereof may be administered to a patient in various ways, e.g. parenterally, topically, enteral, such as oral, rectal or nasal. The compositions will be formulated using adjuvants and diluents suitable for the desired method of administration. Thus the compositions may be administered intravenously as bolus or by continued infusion, intramuscularly - including paravertebrally and periarticularly - subcutaneously, intracutaneously, intra-articularly, intrasynovially, intrathecally, intra-lesionally or periostally.

Parenteral compositions are preferably administered intravenously either in a bolus form or as a constant infusion. For parenteral administration, the inventive compound may be either suspended or dissolved in a sterile vehicle, optionally together with other components, and sterilized before filling into a suitable vial or ampoule and sealing. Adjuvants such as a local anesthetic, preservatives, stabilizers, solution promoters may also and/or buffers be dissolved in the vehicle. The composition may then be frozen and lyophilized to enhance stability. In the case of suspensions, a surfactant or wetting agent and/or other adjuvant as mentioned above may be included in the composition to facilitate uniform distribution of its components.

Compositions formulated for topical administration may, for example, be in aqueous jelly, oily suspension or emulsified ointment form.

Suitable carriers are, in particular, fillers, such as sugars, for example lactose, sucrose, mannitol or sorbitol, cellulose preparations and/or calcium phosphates, for example tricalcium phosphate or calcium hydrogen phosphate, furthermore binders, such as starch paste, using, for example, corn, wheat, rice or potato starch, gelatin, tragacanth, methylcellulose and/or polyvinylpyrrolidone, if desired, disintegrants, such as the abovementioned starches, furthermore carboxymethyl starch, crosslinked polyvinylpyrrolidone, agar, alginic acid or a salt thereof, such as sodium alginate; auxiliaries are primarily glidants, flow-regulators and lubricants, for example silicic acid, talc, stearic acid or salts thereof, such as magnesium or calcium stearate, and/or polyethylene glycol. Sugar-coated tablet cores are provided with suitable coatings which, if desired, are resistant to gastric juice, using, inter alia, concentrated sugar solutions which, if desired, contain gum arabic, talc, polyvinylpyrrolidone, polyethylene glycol and/or titanium dioxide, coating solutions in suitable organic solvents or solvent mixtures or, for the preparation of gastric juice-resistant coatings, solutions of suitable cellulose preparations, such as acetylcellulose phthalate or hydroxypropylmethylcellulose phthalate. Colorants or pigments, for example to identify or to indicate different doses of active ingredient, may be added to the tablets or sugar-coated tablet coatings.

The dose of the active ingredient depends on the warm-blooded animal species, the age and the individual condition and on the manner of administration.

A further embodiment of the invention concerns the use of the inventive compound for the inhibition of reverse transcriptase like, for example, AMV RT, M-MuLV RT, HSV-RT and HIV-RT.

Another embodiment of the invention is the use of the inventive compound in a screen as for example in a screen for the presence or absence of reverse transcriptase like AMV RT, M-MuLV RT, HSV-RT and HIV-RT. In order to determine the amount of modified nucleotide that is inserted, the modified nucleotide may be further modified in order to ease the detection. A suitable modification in this respect is, for example, the insertion of a radioactive atom like ¹⁴C, ³²P, ³H and ³⁵S.

Another embodiment of the invention is the use of the inventive compound for the generation of photolytically cleavable DNA and RNA and analogues thereof. This can be used, for example, to generate linker with a photolytically cleavable restriction site, which are also part of the invention. This linker may be synthesized, e.g., with the aid of PCR techniques and chemically synthesized primer, comprising the inventive compound. The inventive compounds may be used also to generate single-strand gaps or to generate RNA and DNA that can be photolytically connected with a RNA or DNA binding protein as shown below.

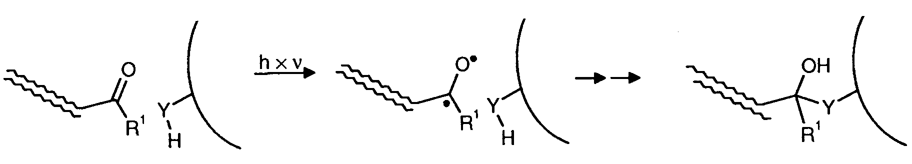

The introduction of the inventive nucleotide into the RNA or DNA may be performed by a reverse transcriptase or by chemically synthesizing oligos that can be elongated with PCR techniques.

Also embraced by the scope of the invention is a process for the synthesis of the inventive compounds. In a preferred embodiment of the invention the process comprises the following steps: 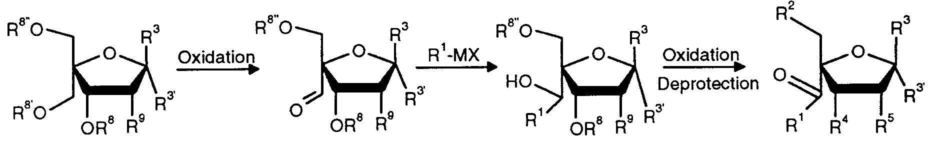 wherein
X, R¹, R², R³, R^{3,}, R⁴ and R⁵ are as defined above;
M is a metal radical that can be used in Grignard reactions like Al, Hg, Mg and Zn;
R⁸, H⁸ 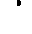 , H⁸ 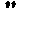 , are. independent of one another, protecting groups; and
R⁹ is hydrogen or OH⁸

Suitable methods for oxidation, Grignard reactions and deprotection are well known to one skilled in the art.

### Examples

The following examples illustrate the invention and should not be construed as a limitation thereof.

### Abbreviations

- DMF: dimethylformamide
- DMSO: dimethylsulfoxide
- DMTrCl: 4,4 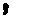 -dimethoxytriphenylmethyl chloride
- DMTr: 4,4 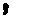 -dimethoxytriphenylmethyl
- DMAP: 4-dimethyl-aminopyridine
- TBAF: tetrabutylammonium fluoride
- TBDMS: tert-butyldimethylsilyl
- TBDPS: tert-butyldiphenylsilyl
- THF: tetrahydrofurane

Example 1: 3'-O-tert-Butyldimethylsilyl-5'-O-tert-butyldiphenylsilyl-2'-deoxy-4'-hydroxy-
methyl-thymidine

2.71 g (3.93 mmol) of a compound with formula 3 (Yang *et al*, Tetrahedron Lett. (1992), **33**, 37-40; Yang *et al*, Tetrahedron Lett. (1992), **33**, 41-44). 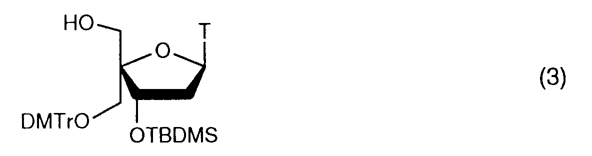 and imidazole (0.80 g, 11.8 mmol) are dissolved in DMF (7 ml) and *tert*-butyldiphenylchlorosilane (1.11 ml, 4.33 mmol) is added at 25°C. After stirring for 24 h at 25°C the reaction mixture is poured onto water (60 ml), extracted three times with CH₂Cl₂ (80 ml each), dried over MgSO₄ and evaporated *in vacuo*. The resulting yellow foam is dissolved in THF (5 ml) and 80% acetic acid (17 ml) is added. After stirring at 25°C for 24 h the reaction mixture is cooled to 0°C and neutralized with a 25% aq. NH₃ soln. (20 ml), poured onto water (80 ml) and extracted twice with CH₂Cl₂ (50 ml each). The combined organic phases are washed with a sat. aq. NaHCO₃ soln. (100 ml), dried over MgSO₄ and evaporated *in vacuo*. Flash chromatography (ethyl acetate/pentane 1:2) gives 1.82 g (74%) of a compound of formula 4 as a pale yellow foam. 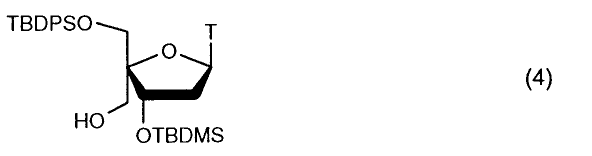
IR (NaCl): 3052, 2955, 2931, 2858, 1693, 1471, 1428, 1264, 1113, 835, 739, 703.
¹H-NMR (CDCl₃): 0.07 (*s*, CH₃―Si), 0.11 (*s*, CH₃―Si), 0.91 (*s*, *t*-Bu―Si), 1.10 (*s*, *t*-Bu―Si), 1.62 (*s*, CH₃―C(5)), 2.27 (*m*, H―C(2b'), OH), 2.36 (*ddd*, *J*=3.0, *J*=6.0, *J*=13.4, H―C(2a')), 3.63 (*dd*, *J*=8.7, *J*=12.1, H―C(5a')), 3.77 (*dd*, *J*=5.0, *J*=12.1, H―C(5b')), 3.80 (*d*, *J*=11.1, H―C(5c')), 3.88 (*d*, *J*=11.1, H―C(5d')), 4.67 (*dd*, *J*=3.0, *J*=6.4, H―C(3')), 6.41 (*dd*, *J*=6.0, *J*=7.8, H― C(1')), 7.42 (*m*, Hₐᵣₒₘ, H―C(6)), 7.66 (*m*, Hₐᵣₒₘ), 9.15 (*s*, NH).
¹³C-NMR (CDCl₃): -5.2 (CH₃―Si), -5.0 (CH₃―Si), 12.0 (*C*H₃―C(5)), 17.9 (Me₃*C*―Si), 19.3 (Me₃*C*―Si), 25.6 ((*C*H₃)₃―C-Si), 27.0 ((*C*H₃)₃―C), 41.8(C(2')), 63.6 (C(5a')), 65.6 (C(5b')), 73.6 (C(3')), 84.2 (C(1')), 89.0 (C(4')), 111.1(C(5)), 127.9-135.5 (Cₐᵣₒₘ, C(6)), 150.3 (C(2)), 163.8 (C(4)).
FAB-MS: 625 (2, [M+1]⁺). Anal. calc. for C₃₃H₄₈N₂O₆Si₂ (624.93):
   C 63.43, H 7.74, N 4.48; found: C 63.31, H 7.83, N 4.49.

### Example 2: 3'-O-tert-Butyldimethylsilyl-5'-O-tert-butyldiphenylsilyl-4'-formyl-thymidin

To a solution of trichloroacetic anhydride (0.40 ml, 2.14 mmol) in CH₂Cl₂ (6 ml) DMSO (0.20 ml, 2.98 mol) is added at -70°C. After stirring for 15 min at -70°C a solution of the compound of formula 4 (0.92 g, 1.46 mmol) in CH₂Cl₂ (2 ml) is added. After stirring for another 30 min at -70°C triethylamine (0.99 ml, 7.01 mmol) is added and the reaction mixture is warmed to 25°C within 30 min. The reaction mixture is poured onto water (60 ml), extracted three times with CH₂Cl₂ (40 ml each), dried over MgSO₄ and evaporated *in vacuo*. Flash chromatography (ethyl acetate/pentane 1:1) gives 0.86 g (95%) of a compound of formula 5 as a pale yellow foam. 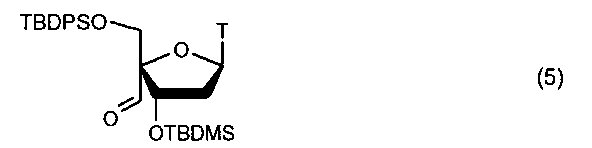
IR (NaCl): 3071, 2931, 2858, 1694, 1472, 1428, 1363, 1281, 1263, 1114, 829.
¹H-NMR (CDCl₃): 0.02 (*s*, CH₃―Si), 0.04 (*s*, CH₃―Si), 0.85 (*s*, *t*-Bu―Si), 1.10 (*s*, *t*-Bu―Si), 1.64 (*s*, CH₃―C(5)), 2.31 (*m*, H―C(2')), 3.91 (*d*, *J*=11.5, H―C(5a')), 4.11 (*d*, *J*=11.5, H―C(5b')), 4.66 (*dd*, *J*=4.8, H―C(3')), 6.70 (*dd*, *J*=8.2, H―C(1')), 7.43 (*m*, Hₐᵣₒₘ), 7.53 (*s*, H―C(6), 7.64 (*m*, Hₐᵣₒₘ), 9.09 (*s*, NH), 9.53 (*s*, C(O)H).
¹³C-NMR (CDCl₃): -5.4 (CH₃―Si), -4.9 (CH₃―Si), 12.0 (*C*H₃―C(5)), 17.9 (Me₃*C*―Si), 19.3 (Me₃*C*―Si), 25.5 (2x(CH₃)₃―C-Si), 41.3(C(2')), 64.5 (C(5')), 76.2 (C(3')), 86.1(C(1')), 92.8 (C(4')), 111.5(C(5)), 127.9-135.5 (Cₐᵣₒₘ, C(6)), 150.3 (C(2)), 163.7 (C(4)), 200.2 (*C*HO).
FAB-MS: 623 (2, [M+1]⁺). Anal. calc. for C₃₃H₄₆N₂O₆Si₂ (622.91):
   C 63.63, H 7.44, N 4.50; found: C 63.28, H 7.40, N 4.39

### Example 3: 3'-O-tert-Butyldimethylsilyl-5'-O-tert-butyldiphenylsilyl-2'-deoxy-4'-(1-hydroxyethyl)-thymidine

To a solution of a compound of formula 5 (0.60 g, 0.96 mmol) in THF (10 ml) a 3 M solution of CH₃MgCl (1.20 ml, 3.60 mmol) in THF is added at 0°C. After stirring for 2 h at 0°C a sat. aq. NH₄Cl soln. (20 ml) is added and extracted three times with CH₂Cl₂ (100 ml each). The combined organic phases are washed with water (100 ml), dried over MgSO₄ and evaporated *in vacuo* to give 0.60 g (97%) of a compound of formula 6 as a colorless foam, which is used in the next step without further purification. 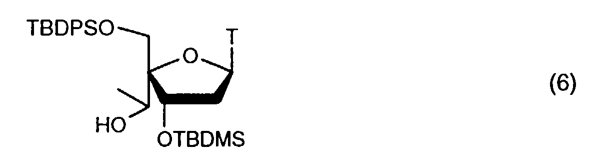
IR (KBr): 3448, 3071, 2954, 2931, 1744, 1686, 1472, 1252, 1228.
¹H-NMR (CDCl₃), diastereomer a: 0.13 (*s*, CH₃―Si), 0.17 (*s*, CH₃―Si), 0.93 (*s*, *t*-Bu―Si), 1.12 (*s*, *t*-Bu―Si), 1.17 (*d*, *J*=6.7, C*H*₃―CHOH), 1.54 (*d*, *J*=1.1, CH₃―C(5)), 2.35 (*m*, H―C(2')), 2.87 (*s*, OH), 4.00 (*d*, *J*=11.5, H―C(5a')), 4.10 (*d*, *J*=11.5, H―C(5b')), 4.14 (*d*, *J*=6.7, *H*―COH), 4.84 (*dd*, *J*=3.8, *J*=6.7, H―C(3')), 6.33 (*dd*, *J*=7.2, H―C(1')), 7.44 (*m*, Hₐᵣₒₘ, H―C(6)), 7.66 (*m*, Hₐᵣₒₘ), 8.52 (*s*, NH), diastereomer b: 0.11 (*s*, CH₃―Si), 0.15 (*s*, CH₃―Si), 0.93 (*s*, *t*-Bu―Si), 1.00 (*s*, *t*-Bu―Si), 1.25 (*d*, *J*=4.7, CH₃―CHOH), 1.61 (*d*, *J*=1.2, CH₃―C(5)), 2.36 (*m*, H―C(2')), 3.38 (*s*, OH), 3.60 (*d*, *J*=11.2, H―C(5a')), 3.80 (*d*, *J*=11.2, H―C(5b')), 3.84 (*d*, *J*=4.7, *H*― COH), 4.84 (*dd*, *J*=2.4, *J*=6.9, H―C(3')), 6.49 (*dd*, *J*=6.0, *J*=8.2, H―C(1')), 7.44 (*m*, Hₐᵣₒₘ, H― C(6)), 7.66 (*m*, Hₐᵣₒₘ), 8.52 (*s*, NH).
¹³C-NMR (CDCl₃), diastereomer a: -5.1 (CH₃―Si), -4.3 (CH₃―Si), 12.0 (*C*H₃―C(5)), 17.1 (*C*H₃―CHOH), 17.9 (Me₃*C*―Si), 19.5 (Me₃*C*―Si), 25.7 ((*C*H₃)₃―C-Si), 27.2 ((*C*H₃)₃―C-Si), 41.6 (C(2')), 63.6 (C(5')), 68.2 (CH₃―*C*HOH), 73.6 (C(3')), 83.6 (C(1')), 89.3 (C(4')), 111.3 (C(5)), 128.0-135.5 (Cₐᵣₒₘ, C(6)), 150.3 (C(2)), 163.7 (C(4)), diastereomer b: - 5.1 (CH₃―Si), -4.5 (CH₃―Si), 12.1 (*C*H₃―C(5)), 16.8 (*C*H₃―CHOH), 18.8 (Me₃*C*―Si), 19.4 (Me₃*C*―Si), 27.1 ((CH₃)₃―C-Si), 27.2 ((*C*H₃)₃―C-Si), 42.7 (C(2')), 65.1 (CH₃―*C*HOH), 69.0 (C(5')), 74.5 (C(3')), 84.6 (C(1')), 90.8 (C(4')), 111.1(C(5)), 128.0-135.6 (Cₐᵣₒₘ, C(6)), 150.2 (C(2)), 163.9 (C(4)).
FAB-MS: 639 (1, [M+1]⁺). Anal. calc. for C₃₄H₅₀N₂O₆Si₂(638.96):
   C 63.91, H 7.89, N 4.38; found: C 63.74, H 8.04, N 4.23.

### Example 4: 4'-Acetyl-3'-O-tert-butyldimethylsilyl-5'-O-tert-butyldiphenylsilyl-thymidine

1,1,1-Triacetoxy-1,1-dihydro-1,2-benzodioxol-3(1*H*)-one (0.43 g, 1.02 mmol) is dissolved in CH₂Cl₂ (7 ml) and a solution of 6 (0.32 g, 0.51 mmol) in CH₂Cl₂ (3 ml) is added at 25°C. After stirring for 1 h the reaction mixture is poured in a mixture of a sat. aq. NaHCO₃ soln. (10 ml) and a sat. aq. Na₂S₂O₃ soln. (50 ml, 1/1, v/v), extracted three times with diethylether (100 ml each), dried over MgSO₄ and evaporated *in vacuo*. Flash chromatography (ethyl acetate/pentane 1:3) gives 0.24 g (75%) of a compound of formula 7 as a colorless foam. 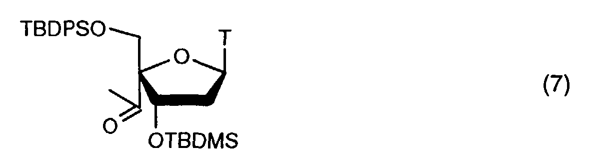
¹H-NMR (CDCl₃): 0.01 (*s*, CH₃―Si), 0.04 (*s*, CH₃―Si), 0.86 (*s*, *t*-Bu―Si), 1.10 (*s*, *t*-Bu―Si), 1.59 (*s*, CH₃―C(5)), 2.28 (*m*, H―C(2'), CH₃―C(O)), 3.95 (*d*, *J*=11.3, H―C(5')), 4.50 (*m*, H―C(3')), 6.66 (*dd*, *J*=6.3, *J*=8.7, H―C(1')), 7.42 (*m*, Hₐᵣₒₘ), 7.61 (*m*, H―C(6), Hₐᵣₒₘ), 8.98 (*s*, NH).
¹³C-NMR (CDCl₃): -5.4 (CH₃―Si), -5.1 (CH₃―Si), 12.0 (*C*H₃―C(5)), 17.9 (Me₃*C*―Si), 19.5 (Me₃*C*―Si), 25.7 ((CH₃)₃―C-Si), 27.1 ((*C*H₃)₃―C-Si), 28.7 (*C*H₃―C(O)), 41.6 (C(2')), 66.7 (C(5')), 76.2 (C(3')), 86.3 (C(1')), 96.3 (C(4')), 111.4 (C(5)), 128.0-135.5 (Cₐᵣₒₘ, C(6)), 150.4 (C(2)), 163.8 (C(4)), 208.8.4 (CH₃―*C*(O)).
FAB-MS: 637 (3, [M+1]⁺). Anal. calc. for C₃₄H₄₈N₂O₆Si₂ (636.94):
   C 64.11, H 7.60, N 4.40; found: C 64.12, H 7.78, N 4.24.

### Example 5: 4'-Acetyl-2'-deoxy-thymidine

To a solution of 7 (0.48 g, 0.75 mmol) in THF (15 ml) a 1M solution of TBAF (1.92 ml, 1.92 mmol) is added at 25°C and stirred for 3 h. Then, silica gel (2 g) is added to the reaction mixture and the solvent is evaporated *in vacuo*. Flash chromatography (ethyl acetate/acetonitrile 4:1) gives 0.18 g (84%) of a compound of formula 7 as a colorless foam. 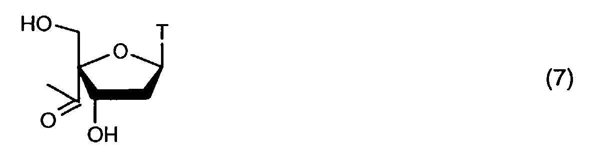
IR (KBr): 3420, 3062, 1701, 1474, 1413, 1357, 1275, 1102, 1054, 1010.
¹H-NMR (CD₃OD): 1.88 (*d*, *J*=1.0, CH₃―C(5)), 2.25 (*s*, CH₃―C(O)), 2.30 (*m*, H―C(2')), 3.86 (*s*, H―C(5')), 4.46 (*m*, H―C(3')), 6.58 (*dd*, *J*=5.9, *J*=9.0, H―C(1')), 7.86 (*d*, *J*=1.0, H―C(6)).
¹³C-NMR (CD₃OD): 12.5 (*C*H₃―C(5)), 28.9 (*C*H₃―C(O)), 41.3 (C(2')), 65.5 (C(5')), 75.5 (C(3')), 87.8 (C(1')), 97.8 (C(4')), 111.8 (C(5)), 138.2 (C(6)), 152.4 (C(2)), 166.4 (C(4)), 211.7 (CH₃― *C*(O)).
FAB-MS: 285 (46, [M+1]⁺). Anal. calc. for C₁₂H₁₆N₂O₆•0.8 H₂0 (298.69):
   C 48.21, H 5.95, N 9.48; found: C 48.18, H 5.90, N 9.12.

### Example 6: 4'-Acetyl-2'-deoxy-5'-O-(4,4'-dimethoxytriphenylmethyl)-thymidine

A mixture of a compound of formula 7 (0.05 g, 0.16 mmol), DMTrCl (2 eq.) and a catalytic amount of DMAP are stirred in pyridine (4 ml/mmol) at 25°C for 24 h. After the reaction is completed, CH₃OH (1 ml/mmol) is added. The reaction mixture is poured onto a sat. aq. NaHCO₃ soln. (100 ml/mmol) and extracted three times with CH₂Cl₂ (50 ml each), dried over MgSO₄ and evaporated *in vacuo*. Flash chromatography (ethyl acetate/pentane/triethylamine 1:1:0.01) gives 0.07 g (72%) of a compound of formula 8 as a pale yellow foam. 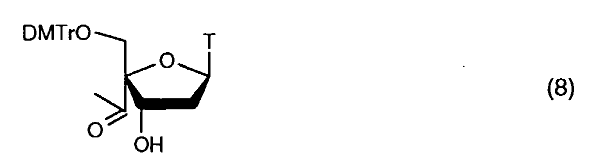
IR (KBr): 3447, 3064, 2955, 1688, 1607, 1509, 1297, 1177, 1092, 1034, 830.
¹H-NMR (CDCl₃): 1.42 (*s*, CH₃―C(5)), 2.30 (CH₃―C(O)), 2.43 (*m*, H―C(2')), 3.12 (s, HO― C(3')), 3.42 (*d*, *J*=9.9, H―C(5a')), 3.55 (*d*, *J*=9.9, H―C(5b')), 3.79 (*s*, CH₃O), 4.65 (*d*, *J*=3.8, H―C(3')), 6.68 (*dd*, *J*=5.7, *J*=8.9, H―C(1')), 6.84 (*m*, Hₐᵣₒₘ), 7.30 (*m*, Hₐᵣₒₘ), 7.56 (*s*, H―C(6)), 9.20 (*s*, NH).
¹³C-NMR (CDCl₃): 11.7 (*C*H₃―C(5)), 28.7 (CH₃―C(O)), 40.1 (C(2')), 55.3 (CH₃―O), 66.4 (C(5')), 75.0 (C(3')), 86.0 (C(1')), 87.6 (CAr₃), 95.7 (C(4')), 111.7 (C(5)), 113.4 (*C*ₐᵣₒₘ―*ortho* to OMe), 127.3-144.0 (C_{arom.} C(6)), 150.5 (C(2)), 158.9 (*C*ᵢₚₛₒ―OMe), 163.8 (C(4)), 211.6 (CH₃― *C*(O)).
FAB-MS: 587 (2, [M+1]⁺). Anal. calc. for C₃₃H₃₄N₂O₈ (586.65):
   C 67.56, H 5.84, N 4.78; found: C 67.22, H 6.37, N 4.29.

### Example 7: O-{3-[4-acetyl-2-deoxy-5-O-(4,4'-dimethoxytriphenylmethyl)-1-(1-thymyl)]-β-D-erythro-pento-1,4-furanosyl}-O-(2-cyanoethyl)-N,N'-diisopropyl-phosphoramidite

The compound of formula 8 (0.06 g, 0.09 mmol), *N,N*-diisopropylethylamine (5.5 eq) and 2-cyanoethyl *N,N*-diisopropylchlorophosphoramidite (2.3 eq) are dissolved in CH₂Cl₂ (4 ml / mmol) and stirred for 2 h at 25°C. The reaction mixture is diluted with CH₂Cl₂ (20 ml) and hydrolyzed with a sat. aq. NaHCO₃ soln. (100 m/mmol), extracted twice with CH₂Cl₂ (50 ml each), dried over MgSO₄ and evaporated *in vacuo*. Flash chromatography (ethyl acetate/pentane/triethylamine 2:1:0.01) gives 0.06 g (78%) of the 3'-O-phosphoramidite of formula 9 as a pale yellow foam. 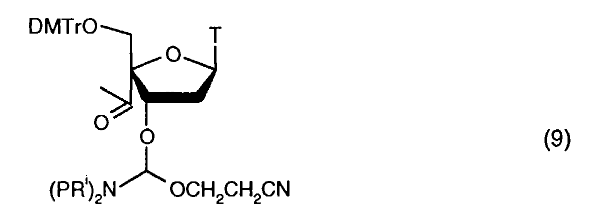
IR (KBr): 3447, 3059, 2967, 2931, 1700, 1654, 1509, 1509, 1466, 1252, 1179, 1032, 831.
¹H-NMR (CDCl₃), mixture of diastereomers: 1.25 (*m*, CH(C*H*₃)₂, CH₃―C(5)), 2.45 (*m*, H-C(2'), CH₂CN, CH₃―C(O)), 3.60 (*m*, CH₂―OP, CH―N, H―C(5'), CH₃O), 4.76 (m, H―C(3')), 6.66 (*m*, H―C(1')), 6.85 (*m*, Hₐᵣₒₘ), 7.31 (*m*, Hₐᵣₒₘ), 7.58 and 7.60 (*d*, *J*=1.0, H―C(6)).
¹³C-NMR (CDCl₃), mixture of diastereomers: 11.7 (*C*H₃―C(5)), 20.3-20.5 (*C*H₂CN), 24.3-24.7 (N(CH(*C*H₃)₂), 28.6 (*C*H₃―C(O)), 39.7 (N(*C*H(CH₃)₂), 43.4 and 43.62 (2*d*, *J*ₐ=12.4, *J*_{b}=12.5, C(2')), 55.3 (CH₃―O), 58.0 and 58.66 (2*d*, *J*ₐ=20.4, *J*_{b}=19.0, CH₂―OP), 65.9 and 66.0 (C(5')), 76.8 (C(3')), 86.0 and 86.1 (C(1')), 87.6 (*C*Ar₃), 94.4 (*d*, *J*=5.8, C(4')), 111.5 (C(5)), 113.4 (*C*ₐᵣₒₘ―*ortho* to OMe), 117.5 (CN), 127.3-144.0 (Cₐᵣₒₘ, (C(6)), 150.3 (C(2)), 158.9 (Cᵢₚₛₒ― OMe), 163.8 (C(4)), 208.0 and 208.2 (CH₃―*C*(O)).
³¹P-NMR (CDCl₃), diastereomer a: 150.1, diastereomer b: 150.0.
FAB-MS: 787 (2, [M+1]⁺). Anal. calc. for C₄₂H₅₁N₄O₉P•0.5 H₂O (795.87):
   C 63.32, H 6.52, N 7.00; found: C 62.97, H 6.50, N 6.85.

Example 8: Solid-phase synthesis of the 4'-modified oligonucleotides (5'-d(T4T*T7))

The synthesis of oligonucleotides of formula 10 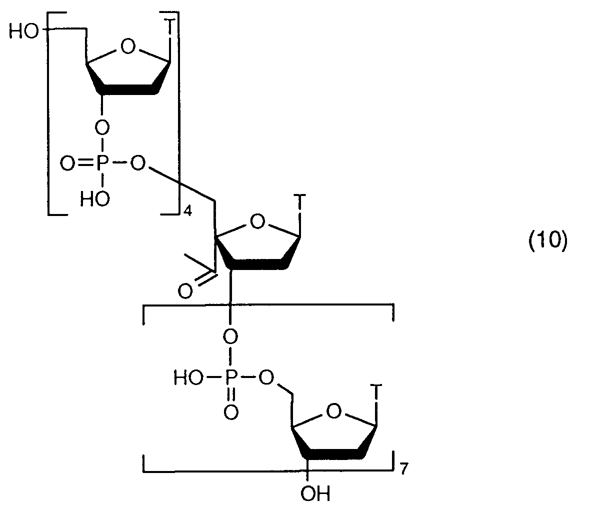 is carried out on an *ABI 392 DNA/RNA* synthesizer in a 1 µmol scale (20 mol equiv. phosphoramidite per cycle, 500 Å CPG support). The standard procedure for β-cyanoethylphosphoramidites is used, except that the coupling time of the modified nucleoside e3 is extended to 30 min. The coupling efficiencies of the modified building blocks e3 are similar to those of the commercially available amidites (98%, assigned by conductivity measurements of the trityl salt released on each cycle). Concentrated NH₃ is used to remove the oligonucleotides from the solid support (55°C, 8 h). The crude oligonucleotides are detritylated and desalted on oligonucleotide cartridges (OPC, *MWG-Biotech*). Preparative HPLC (RP-18, linear gradient of 5-40% acetonitrile (20 min) in 0.1% triethylammonium acetate of pH = 7.0) leads after lyophilyzation to the oligonucleotides 10. MALDI-TOF MS: 3629.2 (calc. 3929.6) (M-H⁺)⁻ for 10.

### Example 9: 3'-O-tert-Butyldimethylsilyl-5'-O-tert-butyldiphenylsilyl-2'-deoxy-4'-(1-hydroxybenzyl)-thymidine

Cul (0.04 g, 0.20 mmol) is suspended in a solution of a compound of formula 5 (0.70 g, 1.12 mmol) in THF (50 ml) at -5°C and a 1 M solution of phenylmagnesium bromide (6.00 ml, 6.00 mmol) is added slowly. The reaction mixture is stirred for 2 h at - 5°C and for 1 h at 0°C. Then, a sat. aq. NH₄Cl soln. (20 ml) is added, the water phase is extracted seven times with diethylether (50 ml each). The organic phase is dried over MgSO₄ and evaporated *in vacuo*. Flash chromatography (ethyl acetate/pentane 1:2) yields 0.62g (79%) of a compound of formula 11 as a colorless foam. 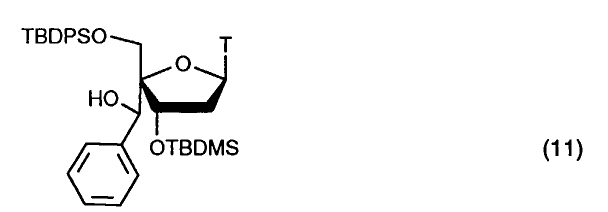
¹H-NMR (CDCl₃): 0.17 (*s*, CH₃―Si), 0.18 (*s*, CH₃―Si), 0.97 (*s*, *t*-Bu―Si), 1.07 (*s*, *t*-Bu―Si), 1.57 (*d*, *J*=1.2, CH₃―C(5)), 2.15 (*m*, H―C(2')), 2.34 (*m*, H―C(2')), 3.26 (*d*, *J*=11.1, H―C(5')), 3.46 (*d*, *J*=11.1, H―C(5')), 4.04 (*d*, *J*=2.7, OH), 4.82 (*d*, *J*=2.9, H―C(3')), 4.94 (*dd*, *J*=7.5, *J*=2.1, H-C(5')), 6.59 (*dd*, *J*=7.8, *J*=6.3, H―C(1')), 7.40 (*m*, C₆H₅ and H―C(6)), 8.6 (*s*, NH).
¹³C-NMR (CDCl₃): -4.7 (CH₃―Si), -4.2 (CH₃―Si), 12.3 (*C*H₃―C(5)), 18.2 ((CH₃)₃*C*―Si) 19.9 ((CH₃)₃*C*―Si), 26.0 ((*C*H₃)₃―C-Si), 27.4 ((*C*H₃)₃―C-Si), 42.5 (C(2')), 66.4 (C(5')), 75.6 (C(5')) 75.9 (C(3')), 85.5 (C(1')), 90.5 (C(4')), 111.3 (C(5)), 128.2-139.0 (C(6), Cₐᵣₒₘ), 150.2 (C(2)), 163.6 (C(4)).
FAB-MS: 701 (1.5, [M+1]⁺). Anal. calc. for C₃₉H₅₂N₂O₅Si₂•0.25 H₂O (703.51):
   C 66.82, H 7.48, N 4.00; found: C 66.82, H 7.47, N 3.84.

### Example 10: 4'-Benzoyl-3'-O-tert-butyldimethylsilyl-5'-O-tert-butyldiphenylsilyl-2'-deoxythymidine

1,1,1-Triacetoxy-1,1-dihydro-1,2-benzodioxol-3(1*H*)-one (0.43 g, 1.02 mmol) is dissolved in CH₂Cl₂ (10 ml) and a solution of a compound of formula 11 (0.46 g, 0.66 mmol) in CH₂Cl₂ (5 ml) is added at 25°C. After stirring for 1 h the reaction mixture is poured in a mixture of a sat. aq. NaHCO₃ soln. (10 ml) and a sat. aq. Na₂S₂O₃ soln (60 ml, 1/1, v/v), extracted three times with diethylether (100 ml each), dried over MgSO₄ and evaporated *in vacuo* to give 0.45 g (99%) of a compound of formula 12 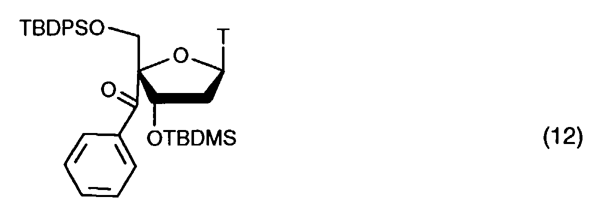 as a colorless foam which is used in the next step without further purification.
¹H-NMR (CDCl₃): -0.06 (*s*, CH₃―Si), 0.06 (*s*, CH₃―Si), 0.74 (*s*, *t*-Bu―Si), 1.61 (*d*, J=1.2, CH₃― C(5)), 2.33 (*m*, H―C(2')), 2.40 (*m*, H―C(2')), 4.21 (*d*, *J*=11.1, H―C(5')), 4.22 (*d*, *J*=11.1, H― C(5')), 4.64 (*d*, *J*=4.8, H―C(3')), 6.64 (*dd*, *J*=9.3, *J*=6.3, H―C(1')), 7.59 (*m*, H―C(6), Hₐᵣₒₘ), 8.16 (*s*, NH).
¹³C-NMR (CDCl₃): -5.2 (CH₃―Si), -4.9 (CH₃―Si), 12.0 (*C*H₃―C(5)), 17.9 (Me₃*C*―Si), 19.4 (Me₃*C*―Si), 25.6 ((CH₃)₃―C-Si), 27.0 ((*C*H₃)₃―C-Si), 41.7 (C(2')), 68.4 (C(5')), 77.2 (C(3')), 86.5 (C(1')), 98.5 (C(4')), 110.9 (C(5)), 127.9-135.6 (C(6), Cₐᵣₒₘ), 150.0 (C(2)), 163.4 (C(4)), 201.4 (C(5')).
FAB-MS: 699 (2.6, [M+1]⁺). Anal. calc. for C₃₉H₅₀N₂O₆Si (698.32):
   C 66.50, H 7.17, N 3.98; found: C 66.45, H 7.11, N 4.04.

### Example 11: 4'-Benzoyl-2'-deoxy-thymidine

To a solution of a compound of formula 12 (6.50 mg, 0.01 mmol) in THF (2 ml) a 1 M solution of TBAF (0.01 ml, 0.01 mmol) is added at 0°C. After stirring for 1 h at 0°C the reaction mixture is directly chromatographed (dichloromethane/methanol 16:1) to remove TBAF. After evaporation *in vacuo*, the residue is dissolved in THF (2 ml) and pyridinium polyhydrogen fluoride (0.07 ml, 70% HF) is added at 25°C. After stirring for 2 d the reaction mixture is poured onto a sat. aq. NaHCO₃ soln. (25 ml), extracted four times with ethyl acetate (50 ml each), dried over MgSO₄ and evaporated *in vacuo*. Flash chromatography (dichloromethane/methanol 16:1) gives 0.60 mg (18%) of a compound of formula 13.

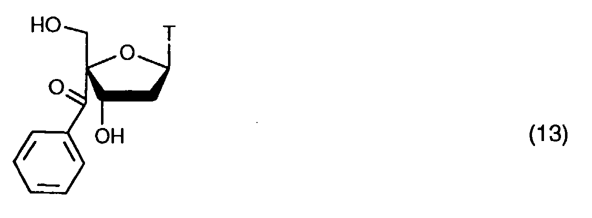

### Example 12: 3'-O-tert-Butyldimethylsilyl-2'-deoxy-5'-C-phenyl-thymidine

CeCl₃ • 7 H₂O (0.63 g, 1.70 mmol) is placed in a 100 ml three-necked flask and heated under stirring at 140°C *in vacuo* (0.1 Torr) for 10 h. After cooling to 0°C, THF (10 ml) is added and stirring is continued for 15 h. The mixture is then agitated at - 5°C for 10 min and a solution of aldehyde of formula 14 (Yang *et al*., Tetrahedron Lett. (1992), **33**, 41) 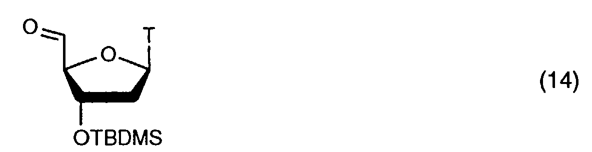 (0.20 g, 0.56 mmol) in THF (10 ml) is added. Stirring at -5°C is continued for 15 min. Phenylmagnesium bromide (1.7 ml of a 1 M solution in THF, 1.70 mmol) is added slowly to the resulting suspension whereupon the color of the solution changes from white to yellow. After 2 h at -5°C and 1 h at 10°C, the mixture is quenched with a sat. aq. NH₄Cl soln. (25 ml), the resulting pale yellow suspension is poured onto water (100 ml) and extracted three times with diethylether (100 ml each), dried over MgSO₄ and evaporated *in vacuo*. Flash chromatography (dichloromethane/ethanol 20:1) gives 0.20 g (82%) of a diastereomeric mixture of the compound of formula 15 as a colorless foam.

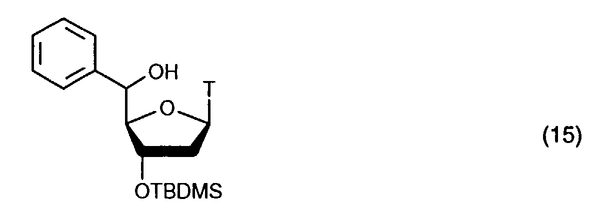

An alternative procedure to synthesize the compound of formula 15 is:
The aldehyde of formula 4 (2.00 g, 5.60 mmol) is dissolved in THF (80 ml) and cooled to -5°C. Cu (0.21 g, 1.10 mmol) is added to the resulting colorless solution, the mixture is agitated for 10 min and phenylmagnesium bromide (3.40 ml of a 1 M solution in THF, 3.40 mmol) is added. After stirring for 2 hours at -5°C the reaction mixture is hydrolyzed with a sat. NH₄Cl soln. (100 ml) and extracted eight times with diethylether (100 ml each), dried over MgSO₄ and evaporated *in vacuo*. Flash chromatography (methylene chloride/ethanol 20:1) gives 1.90 g (78%) of a diastereomeric mixture of the compound of formula 15 as a colorless foam.
¹H-NMH (CDCl₃), diastereomer a: 0.02 (*s*, CH₃―Si), 0.86 (*s*, CH₃―Si), 1.92 (*s*, *t*-Bu―Si), 1.92 (*s*, CH₃―C₅), 2.13-2.21 (*ddd*, *J*=2.8, *J*=5.5, *J*=10.3, H―C(2b')), 2.30-2.39 (*m*, H―C(2a')), 3.43 (*d*, *J*=4.9, HO―C(5')), 4.08 (*dd*, *J*=3.3, *J*=3.8 H―C(4')), 4.52 (*m*, H―C(3')), 4.81 (*dd*, *J*=3.8, J=4.9, H―C(5')), 6.11 (*dd*, *J*=5.5, *J*=8.0, H―C(1')), 7.28-7.52 (*m*, Hₐᵣₒₘ, H―C(6)), 9.24 (s, NH)) diastereomer b: 0.09 (*s*, CH₃―Si), 0.10 (*s*, CH₃―Si), 0.90 (*s*, *t*-Bu―Si), 1.01 (*s*, *t*-Bu―C), 1.93 (*d*, *J*=1.2, CH₃―C(5)), 2.05-2.22 (*m*, H―C(2b')), 2.36-2.38 (*m*, H―C(2a')), 2.82-2.83 (*m*, HO― C(5')), 3.50 (*m*, H―C(5')), 4.07 (*t*, *J*=2.2, H―C(4')), 4.61 (*dt*, *J*=5.7, *J*=2.1, H―C(3')), 6.09 (*dd*, *J*=8.7, *J*=5.8, H―C(1')), 7.28-7.52 (*m*, Hₐᵣₒₘ, H―C(6)), 8.22 (s, NH).
¹³C-NMR (CDCl₃), diastereomer a: -4.7 (CH₃―Si), -4.6 (CH₃―Si), 12.5 (*C*H₃―C(5)), 18.0 (Me₃*C*―Si), 26.5 ((*C*H₃)₃―C), 39.7 (C(2')), 74.3 (C(5')), 78.3 (C(3')), 86.2 (C(1')), 87.1 (C(4')), 111.0 (C(5)), 125.9-128.8 (Cₐᵣₒₘ), 137.4 (C(6)), 150.6 (C(2)), 164.1 (C(4)), diastereomer b: - 4.9 (CH₃―Si), -4.0 (CH₃―Si), 12.4 (*C*H₃―C(5)), 17.9 (Me₃*C*―Si), 25.7 ((*C*H₃)₃―C-Si), 40.7 (C(2')), 72.4 (C(5')), 80.5 (C(3')), 86.0 (C(1')), 88.7 (C(4')), 110.9 (C(5)), 125.9-128.8 (Cₐᵣₒₘ), 136.9 (C(6)), 150.4 (C(2)), 163.95 (C(4)).
FAB-MS: 433 (11, [M+1]⁺). Anal. calc. for C₂₂H₃₂N₂O₅Si (432.60):
   C 61.08, H 7.46, N 6.48; found: C 60.76, H 7.70, N 6.48.

### Example 13: 1-(3-O-tert-butyldimethylsilyl-2-deoxy-5-oxo-5-C-phenyl-β-D-erythro-pento-1,4-furanosyl)-thymine

To a solution of trifluoroacetic anhydride (0.30 ml, 2.23 mmol) in CH₂Cl₂ (5 ml), DMSO (0.30 ml, 4.14 mmol) in CH₂Cl₂ (4 ml) is added dropwise at -60°C. The mixture is stirred for 30 min at -60°C. Then, a solution of compound of formula 15 (0.27 g, 0.62 mmol) in CH₂Cl₂ (10 ml) is added slowly. Agitation for one hour at -60°C is followed by the addition of NEt₃ (0.5 ml, 165 mmol). The reaction mixture is diluted with CH₂Cl₂ (20 ml) hydrolyzed with a sat. aq. NaHCO₃ soln. (50 ml), extracted four times with CH₂Cl₂ (75 ml each), dried over MgSO₄ and evaporated *in vacuo*. Flash chromatography (pentane/ether/dichloromethane 3:3:4) yields 0.23 g (85%) of a compound of formula 16 as a colorless foam. 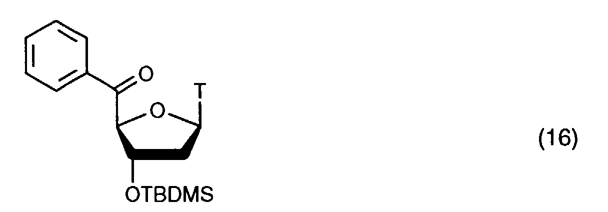
IR (KBr): 3325, 3185, 3065, 2954, 2923, 2855, 1695, 1471, 1278, 1218, 1133, 1085, 982, 837, 777, 692.
¹H-NMR (CDCl₃): 0.07 (*s,* CH₃―Si), 0.08 (*s*, CH₃―Si), 0.95 (*s*, *t*-Bu―Si), 2.02 (*s*, CH₃―C(5)), 2.01 (*ddd, J*=3.5, *J*=5.5, *J*=11.6, H-C(2b')), 2.35 (*dd*, *J*=5.4, *J*=11.6, H―C(2a')), 4.57 (*d*, *J*=5.4, H―C(3')), 5.45 (*d*, *J*=1.4, H―C(4')), 6.59 (*dd*, *J*=5.4, *J*=8.8, H―C(1')), 7.55 (*t*, *J*=7.7, *m*-Hₐᵣₒₘ), 7.68 (*t*, *J*=7.1, *p*-Hₐᵣₒₘ), 8.02 (*d*, *J*=7.2, *o*-Hₐᵣₒₘ), 8.23 (s, H―C(6)).
¹³C-NMR (CDCl₃): -4.99 (CH₃―Si), -4.64 (CH₃―Si), 12.69 (*C*H₃―C(5)), 17.83 (Me₃*C*―Si), 25.60 ((*C*H₃)₃―C-Si), 39.99 (C(2')), 74.63 (C(3')), 86.12 (C(1')), 87.60 (C(4')), 111.2 (C(5)), 128.5-134.5 (Cₐᵣₒₘ), 136.2 (C(6)), 150.4 (C(2)), 163.8 (C(4)), 197.0 (Ph*C*O). FAB-MS: 431 (31, [M+1]⁺). Anal. calc. for C₂₂H₃₀N₂O₅Si (430.57): C 61.37, H 7.02, N 6.51; found: C 61.05, H 7.12, N 6.36.

### Example 14: 1-(2-deoxy-5-C-phenyl-5-oxo-β-D-erythro-pento-1,4-furanosyl)-thymine

A solution of the compound of formula 16 (0.15 g, 0.36 mmol) in THF (10 ml) is treated with a 1 M solution of TBAF (0.40 ml, 0.40 mmol) in THF at 0°C. After stirring for 45 min at 0°C the reaction mixture is directly chromatographed without previous work up. Flash chromatography (dichloromethane/methanol 20:1) gives 0.10 g (89%) of a compound of formula 17 as a colorless foam. 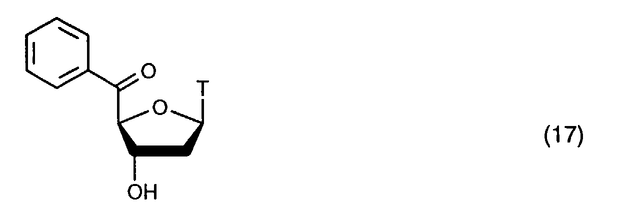
IR (KBr): 3325, 3185, 3065, 2954, 2923, 2855, 1695, 1471, 1278, 1218, 1133, 1085, 982, 837, 777, 692.
¹H-NMR (CDCl₃+10% CD₃OD): 1.93 (*ddd*, *J*=2.8, *J*=5.0, *J*=13.6, H―C(2b')), 2.01 (*s*, CH₃― C(5)), 2.42 (*dd*, *J*=5.2, *J*=13.6, H―C(2a')), 4.59 (*d*, *J*=5.0, H―C(3')), 5.52 (*d*, *J*=1.1, H―C(4')), 6.58 (*dd*, *J*=5.0, *J*=9.3, H―C(1')), 7.55 (*dd*, *J*=7.3, *J*=7.6, *m*-Hₐᵣₒₘ), 7.67 (*t*, *J*=7.4, *p*-Hₐᵣₒₘ), 8.07 (*d*, *J*=7.2, *o*-Hₐᵣₒₘ), 8.37 (s, H―C(6)).
¹³C-NMR (CDCl₃ +10% CD₃OD): 12.27 (CH₃―C(5)), 39.04 (C(2')), 73.47 (C(3')), 86.02 (C(1')), 87.45 (C(4')), 111.0 (C(5)), 128.1-134.3 (Cₐᵣₒₘ), 136.6 (C(6)), 150.1 (C(2)), 164.5 (C(4)), 197.1 (Ph*C*O).
FAB-MS: 317 (15, [M+1]⁺). Anal. calc. for C₁₆H₁₆N₂O₅ (316.32):
   C 60.75, H 5.10, N 8.86; found: C 61.19, H 5.48, N 8.55.

### Example 15: 4'-C-Benzoyl-2'-deoxy-thymidine, 1-(4-benzoyl-2-deoxy-α-L-threo-pento-1,4-furanosyl)-thymine, and 1-(4-benzoyl-2-deoxy-β-D-threo-pento-1,4-furanosyl)-thymine

Ba(OH)₂•8 H₂O (0.12g, 0.68 mmol) is suspended in a solution of a compound of formula 17 (0.18 g, 0.56 mmol) in dioxane/water 10:1 (3.6 ml). After addition of a 36% aq. form-aldehyde soln. (0.30 ml, 3.60 mmol), the mixture is sonicated for 30 sec (sonication bath). Stirring at 25°C is continued for 1 h. Then, the reaction mixture is frozen with liquid N₂ and lyophylized *in vacuo*. Flash chromatography of the white residue (dichloromethane/methanol 20:1) gives 38 mg (22 %) of a compound of formula 19 and 126 mg (73%) of the mixture of compounds of formulae 18:13 (2.5:1) as colorless crystals.

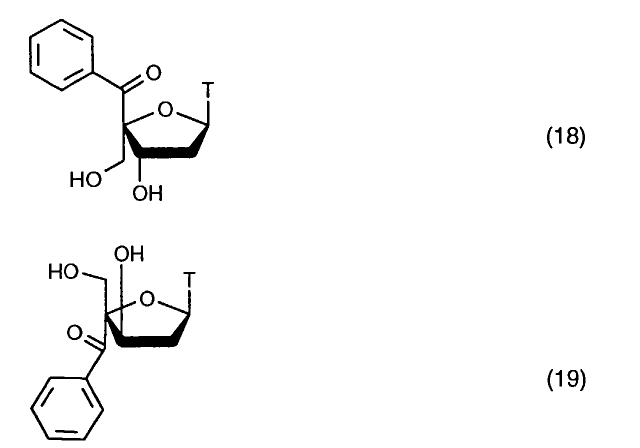

Prep. HPLC (*Knauer*, RP-18, 7µm; length 250mm, diameter 15mm; flow: 6ml/min; H₂O/CH₃CN = 81:19) gives 38 mg compound of formula 13 (22%) and 72 mg compound of formula 18 (42%) as colorless crystals.

### Data for compound of formula 13:

IR (KBr): 3442, 3065, 2935, 1692, 1633, 1512, 1141, 1042, 953, 752.
¹H-NMR (CDCl₃ + 10% CD₃OD):1.92 (*s*, CH₃―C(5)), 2.39 (*ddd*, *J*=1.5, *J*=5.6, *J*=9.0, H-C(2b')), 2.47 (*ddd*, *J*=4.0, *J*=5.2, *J*=9.0, H―C(2a')), 3.96 (*d*, *J*=11.6, H―C(5b')), 4.06 (*d*, *J*=11.6, H―C(5a')), 4.94 (*m*, H―C(3')), 6.46 (*dd*, *J*=4.0*, J*=5.6, H―C(1')), 7.45 (*t*, *J*=7.9, *m*-Hₐᵣₒₘ), 7.51 (*t*, *J*=6.7, *p*-Hₐᵣₒₘ), 7.82 (*s*, H―C(6)), 7.92 (*d*, *J*=7.2, *o*-Hₐᵣₒₘ). NOE (¹H-NMR DMSO-*d*₆): (irradiated H → affected H; ++ = strong, + = medium, (+) = weak): H―C(6)→H―C(5')(+) and H―C(1')(++); *o*-Hₐᵣₒₘ→H―C(5')(+) and H―C(1')((+)); H―C(1')→ *o*-Hₐᵣₒₘ(+) and H―C(6)(+); H―C(3')→H-C(5')(+); H―C(5')→*o*-H-Ph(+) and H―C(6)(+) and H―C(3'); H―(C2'a)→H―C(6)(+).
¹³C-NMR (CDCl₃ + 10% CD₃OD): 12.35 (*C*H₃―C(5)), 40.07 (C(2')), 65.31 (C(5')), 74.19 (C(3')), 85.12 (C(1')), 97.67 (C(4')), 109.6 (C(5)), 127.8-136.1 (Cₐᵣₒₘ), 138.3 (C(6)), 150.5 (C(2)), 163.7 (C(4)), 202.3 (Ph*C*O).
FAB-MS: 347 (33, [M+1]⁺). Anal. calc. for C₁₇H₁₈N₂O₆·0.75 H₂O (359.85):
   C 56.67, H 5.60, N 7.78; found: C 56.61, H 5.54, N 7.74.

### Data for compound of formula 18:

IR (KBr): 3454, 3041, 2899, 1701, 1687, 1537, 1049, 953, 752, 672.
¹H-NMR (DMSO-*d*₆): 1.36 (*s*, CH₃―C(5)), 2.03 (*m*, H―C(2b'), H―C(2a')), 3.84 (*dd*, *J*=3.3, *J*=11.6, H―C(5b')), 4.00 (*dd*, *J*=4.9, *J*=11.6, H―C(5a')), 4.75 (*m*, H―C(3')), 5.13 (*m*, OH), 5.75 (*m*, -OH), 6.39 (*dd*, *J*=6.2, *J*=8.5, H―C(1')), 6.59 (*s*, H―C(6)), 7.44 (*t*, *J*=7.9, *m*-Hₐᵣₒₘ), 7.56 (*t*, *J*=7.3, *p*-Hₐᵣₒₘ), 7.92 (*d*, *J*=7.1, *o*-Hₐᵣₒₘ), 11.2 (s, -NH-).
NOE (¹H-NMR DMSO-*d*₆): (irradiated H → affected H; ++ = strong, + = medium, (+) = weak): *o*-Hₐᵣₒₘ→H―C(6)(+); H―C(6)→H―C(3')(+) and *o*-Hₐᵣₒₘ((+)) and H―C(1')(++); H―C(1')→H-C(5')((+)); H―C(3')→H-C(6)((+)); H-C(5')→*o*-H_{arom.} (+) and H―C(1')((+)) and HO―C(3')(+).
¹³C-NMR (DMSO-*d*₆):12.07 (CH₃―C(5)), 38.22 (C(2')), 64.65 (C(5')), 74.45 (C(3')), 84.92 (C(1')), 97.00 (C(4')), 108.9 (C(5)), 127.7-135.7 (Cₐᵣₒₘ), 138.8 (C(6)), 150.5 (C(2)), 163.4 (C(4)), 203.7 (Ph*C*O).
FAB-MS: 347 (32, [M+1]⁺). Anal. calc. for C₁₇H₁₈N₂O₆•0.75 H₂O (359.85):
   C 56.67, H 5.60, N 7.78; found: C 56.55, H 5.68, N 7.71.

### Data for compound of formula 19:

IR (KBr): 3418, 3066, 2929, 1685, 1596, 1279, 1104, 1052, 953, 786, 693.
¹H-NMR (CDCl₃ + 10% CD₃OD):1.95 (*s*, CH₃―C(5)), 2.06 (*ddd*, *J*=2.6, *J*=3.9, *J*=14.8, H― C(2b')), 2.49 (*dd*, *J*=5.8, *J*=14.8, H―C(2a')), 4.21 (*d*, *J*=11.8, H―C(5b')), 4.29 (*d*, *J*=11.8, H― C(5a')), 4.94 (*m*, H―C(3')), 6.07 (*dd*, *J*=3.9, *J*=8.0, H―C(1')), 7.45 (*dd*, *J*=7.7, *J*=8.0, *m*-Hₐᵣₒₘ), 7.56 (*t*, *J*=7.5, *p*-Hₐᵣₒₘ), 7.95 (*s*, H―C(6)), 8.12 (*d*, *J*=7.2, *o*-Hₐᵣₒₘ).
NOE (¹H-NMR DMSO-*d*₆): (irradiated H→affected H; ++ = strong, + = medium, (+) = weak): *o*-H_{*arom*}→H―C(5')(+) and H―C(1')((+)); H―C(6)→H―C(5')(+) and H―C(1')(++); H―C(1')→ *o*-Hₐᵣₒₘ (+) and H-C(6) (+); H―C(3')→*o*-Hₐᵣₒₘ ((+)) and H―C(1') ((+)); H―C(5') → *o*-Hₐᵣₒₘ (+) and H―C(6) ((+)); H―C(2'a)→H-C(5') (+).
¹³C-NMR (CDCl₃ + 10% CD₃OD):12.48 (*C*H₃―C(5)), 40.07 (C(2')), 63.94 (C(5')), 72.36 (C(3')), 83.20 (C(1')), 98.19 (C(4')), 109.3 (C(5)), 128.1-136.2 (Cₐᵣₒₘ), 136.8 (C(6)), 150.4 (C(2)), 163.6 (C(4)), 195.3 (Ph*C*O).
FAB-MS: 347 (59, [M+1]⁺). Anal. calc. for C₁₇H₁₈N₂O₆•0.75 H₂O (359.85):
   C 56.67, H 5.60, N 7.78; found: C 56.57, H 5.52, N 7.75.

Example 16: 4'-Benzoyl-2'-deoxy-5'-O-(4,4'-dimethoxytriphenylmethyl)-thymidine

Compound of formula 13 (38 mg, 0.109 mmol) is converted into 56 mg (80%) of a compound of formula 20 as described in example 6 and purified using flash chromatography (ethyl acetate/pentane/triethylamine 1:3:0.01).
IR (KBr): 3392, 3059, 2954, 2835, 1685, 1607, 1508, 1465, 1446, 1278, 1251, 1177, 1074, 1033, 829, 696. 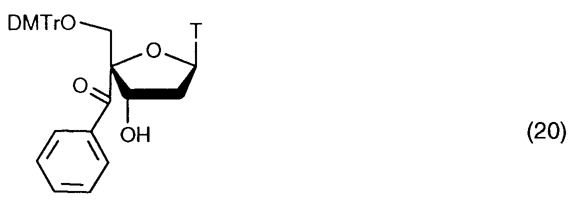
¹H-NMR (CDCl₃): 1.50 (*s*, CH₃―C(5)), 2.40 (*m*, H―C(2'b), H―C(2'a)), 3.55 (*d*, *J*=9.9, H― C(5'b)), 3.69 (*d*, *J*=9.9, H―C(5'a)), 3.78 (*s*, CH₃O), 4.75 (*dd*, *J*=1.9, *J*=3.6, H―C(3')), 6.51 (*dd*, *J*=5.7, *J*=8.8, H―C(1')), 6.79-7.45 (*m*, Hₐᵣₒₘ), 7.50 (*s*, H―C(6)), 7.96 (*d*, *J*=6.2, *o*-Hₐᵣₒₘ).
¹³C-NMR (CDCl₃): 11.86 (CH₃―C(5)), 39.08 (C(2')), 55.22 (CH₃―O), 67.61 (C(5')), 75.55 (C(3')), 85.81 (C(1')), 87.95 (*C*Ar₃), 95.92 (C(4')), 111.4 (C(5)), 113.3 (*C*ₐᵣₒₘ―*ortho* to OMe), 127.2-143.8 (Cₐᵣₒₘ), 135.4 (C(6)), 150.2 (C(2)), 158.7 (*C*ᵢₚₛₒ―OMe), 163.6 (C(4)), 202.8 (Ph*C*O).
FAB-MS: 648 (1.7, [M+1]⁺). Anal. calc. for C₃₈H₃₆N₂O₈•0.5 H₂O (657.72):
   C 69.12, H 5.62, N 4.26; found: C 69.02, H 5.55, N 4.18.

### Example 17: O-{3-[4-benzoyl-2-deoxy-5-O-(4,4'-dimethoxytriohenylmethyl)-1-(1-thymyl)]-β-D-erythro-pento-1,4-furanosyl}-O-(2-cyanoethyl)-N,N'-diisopropyl-phosphoramidite

The compound of formula 20 (50 mg, 0.077 mmol) is converted into 50 mg (76%) of a compound of formula 21 as described in example 7 and purified using flash chromatography (ethyl acetate/pentane/triethylamine 2:1:0.01). 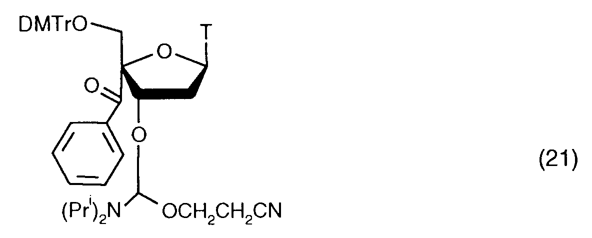
IR (KBr) mixture of diastereomers: 3177.8, 3066.7, 2966.7, 1686.3, 1605.6, 1511.1, 1466.7, 1250.0, 1177.8, 1122.2, 1072.2, 1033.3, 827.8, 755.6, 700.0.
¹H-NMR (CDCl₃), diastereomer a: 1.17 (*m*, CH(C*H*₃)₂), 1.48 (*s*, CH₃―C(5)), 2.42 (*m*, CH₂CN), 2.65 (*dd*, *J*=5.0, *J*=13.2, H―C(2b'), 2.78 (*dd*, *J*=3.7, *J*=13.2, H―C(2a'), 3.45 (*m*, CH₂―OP, CH―N, H―C(5'), CH₃O), 4.90 (*dd*, *J*=4.6, *J*=8.8, H―C(3')), 6.60 (*dd*, *J*=3.7, *J*=5.0, H―C(1')), 6.83 (*m*, Hₐᵣₒₘ), 7.12-7.52 (*m*, Hₐᵣₒₘ), 7.66 (*s*, H―C(6)), 7.82 (*d*, *J*=7.1, *o*-Hₐᵣₒₘ), 8.67 (s, NH), diastereomer b: 1.17 (*m*, CH(C*H*₃)₂), 1.55 (*s*, CH₃―C(5)), 2.37 (*m*, CH₂CN, H―C(2'), 3.61 (*m*, CH₂―OP, CH―N, H―C(5'), CH₃O), 5.00 (*dd*, *J*=4.1, *J*=11.9, H―C(3')), 6.68 (*dd*, *J*=2.3, *J*=7.7, H―C(1')), 6.75 (*m*, Hₐᵣₒₘ), 7.14-7.61 (*m*, Hₐᵣₒₘ), 7.62 (*s*, H―C(6)), 8.09 (*d*, *J*=7.3, *o*-Hₐᵣₒₘ), 8.67 (s, NH).
¹³C-NMR (CDCl₃). diastereomer a: 11.70 (*C*H₃―C(5)), 20.19 (*d*, *J*=7.1, *C*H₂CN), 24.13 (*d*, *J*=7.0, N(CH(*C*H₃)₂), 24.29 (*d*, *J*=7.0, N(CH(*C*H₃)₂), 43.54 (*d*, J=12.5, N(*C*H(CH₃)₂), 47.64 (*d*, *J*=7.0, C(2')), 55.24 (CH₃―O), 58.50 (d, *J*=18.9, CH₂―OP), 67.33 (C(5')), 77.50 (*d*, *J*=18.9, C(3')), 85.83 (C(1')), 87.69 (*C*Ar₃), 96.21 (*d*, *J*=6.1, C(4')), 112.9 (C(5)), 113.3 (Cₐᵣₒₘ *ortho* to OMe), 117.5 (CN), 127.1-143.9 (Cₐᵣₒₘ), 135.7 (C(6)), 150.2 (C(2)), 158.7 (*C*ᵢₚₛₒ―OMe), 163.7 (C(4)), 199.5 (Ph-*C*O), diastereomer b: 12.35 (*C*H₃―C(5)), 19.94 (*d, J*=7.8, *C*H₂CN), 24.18 (*d*, *J*=7.2, N(CH(*C*H₃)₂), 24.39 (*d*, *J*=7.2, N(CH(*C*H₃)₂), 43.49 (*d*, J=12.9, N(*C*H(CH₃)₂), 47.41 (*d*, *J*=7.2, C(2')), 55.18 (CH₃-O), 58.35 (*d*, *J*=19.1, CH₂―OP), 67.33 (C(5')), 77.47 (*d*, *J*=19.2, C(3')), 85.65 (C(1')), 87.69 (*C*Ar₃), 96.52 (*d*, *J*=6.3, C(4')), 111.1 (C(5)), 113.3 (Cₐᵣₒₘ *ortho* to OMe), 117.5 (CN), 127.1-143.9 (Cₐᵣₒₘ), 135.6 (C(6)), 150.1 (C(2)), 158.4 (*C*ᵢₚₛₒ― OMe), 163.8 (C(4)), 200.1 (Ph-*C*O).
³¹P-NMR (CDCl₃), diastereomer a: 149.8, diastereomer b: 149.4.
FAB-MS mixture of diastereomers: 849 (1.1, [M+1]⁺). Anal. calc. for mixture of diastereomers C₄₇H₅₃N₄O₉P·H₂O (866.49):
   C 65.60, H 6.39, N 6.46; found: C 65.57, H 6.42, N 6.61.

### Example 18: 3'-O-tert-Butyldimethylsilyl-5'-O-tert-butyldiphenylsilyl-2'-deoxy-4'-(1-hydroxypropyl)-thymidine

To a solution of a compound of formulas (62 mg, 0.10 mmol) in THF (2 ml) a 1.5 M solution of EtMgBr (0.3 ml, 0.45 mmol) in ether is added at -78°C. After stirring for 1.5 h at - 78°C a sat. aq. NH₄Cl soln. is added (20 ml) and extracted three times with CH₂Cl₂ (50/20/20 ml). The combined organic phases are washed with water (20 ml), dried over MgSO₄, and evaporated *in vacuo*. Flash-chromatography (pentane : acetone 3:1) yields 41 mg (63 %) diastereomer a of formula 22 as a colorless glass. Diastereomer b is obtained as a mixture with an Isomer, where the TBDMS group has moved from the 3'-position (12 mg, 18 %, ratio (22) : Isomer= 1 : 2). 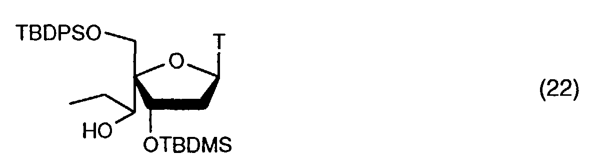
IR (KBr): 3323, 3233, 3074, 2954, 2931, 2893, 2857, 1718, 1702, 1683, 1472, 1428, 1273, 1205, 1086, 1036, 832, 778, 710, 700, 505.
¹H-NMR (CDCl₃): diastereomer a: 0.12 (*s*, CH₃―Si), 0.16 (*s*, CH₃―Si), 0.93 (*s*, *t*-Bu―Si), 0.96 (*t*, *J*=7.2, CH₃), 1.10 (*s*, *t*-Bu―Si), 1.20 (*m*, C*H*₂(a)―CHOH), 1.54 (*d*, *J*=1.2, CH₃―C(5)), 1.66 (*m*, C*H*₂(b)―CHOH), 2.30 (*m*, H―C(2a')), 2.38 (*ddd*, *J*=3.9, *J*=6.3, *J*=13.4, H―C(2b')), 2.80 (*dd*, ^{*4*}*J*=1.2, *J*=4.1, OH), 3.78 (*ddd*, *J*=2.0, *J*=4.1, *J*=10.8, *H*―COH), 3.96 (*d*, *J*=11.4, H― C(5a')), 4.10 (*d*, *J*=11.4, H―C(5b')), 4.81 (*dd*, *J*=3.9, *J*=6.3, H―C(3')), 6.31 (*dd*, *J*=6.3, *J*=7.3, H―C(1')), 7.42 (*m*, Hₐᵣₒₘ, H―C(6)), 7.65 (*m*, Hₐᵣₒₘ), 8.96 (*s*, NH). diastereomer b: 0.09 (*s*, CH₃―Si), 0.13 (*s*, CH₃―Si), 0.91 (*s*, *t*-Bu―Si), 0.94 (*t*, *J*=7.2, CH₃), 1.09 (*s*, *t*-Bu―Si), 1.40 (*m*, C*H*₂―CHOH), 1.57 (*s*, CH₃―C(5)), 2.30 (*m*, H―C(2')), 3.03 (*dd*, *J*=1.5, *J*=2.9, OH), 3.67 (*d*, *J*=11.0, H―C(5a')), 3.85 (*d*, *J*=11.2, H―C(5b')), 3.95 (*m*, *H*―COH), 4.75 (*dd*, *J*=2.7, *J*=6.8, H― C(3')), 6.45 (*dd*, *J*=6.1, *J*=8.1, H―C(1')), 7.40 (*m*, Hₐᵣₒₘ, H―C(6)), 7.64 (*m*, Hₐᵣₒₘ), 8.46 (*s*, NH).
¹³C-NMR (CDCl₃), diastereomer a: -5.1 (CH₃―Si), -4.4 (CH₃―Si), 11.3 (*C*H₃―CH₂), 11.9 (*C*H₃―C(5)), 17.8 (Me₃*C*―Si), 19.4 (Me₃*C*―Si), 23.7 (*C*H₂―CHOH), 25.7 ((*C*H₃)₃―C-Si), 27.1 ((*C*H₃)₃₋C-Si), 41.5 (C(2')), 63.6 (C(5')), 73.5, 73.8 (Et-*C*HOH, C(3')), 83.4 (C(1')), 89.4 (C(4')), 111.3 (C(5)), 127.9, 128.0 (m-Cₐᵣₒₘ), 130.0, 130.2 (p-Cₐᵣₒₘ), 132.3, 132.9 (i-Cₐᵣₒₘ), 135.1 (C(6)), 135.2, 135.4 (o-Cₐᵣₒₘ), 150.3 (C(2)), 163.7 (C(4)).
FAB-MS: 653 (3, [M+1]⁺).

### Example 19: 4'-Propionyl-3'-O-tert-butyldimethylsilyl-5'-O-tert-butyldiphenylsilyl-thymidine

1,1,1-Triacetoxy-1,1-dihydro-1,2-benzodioxol-3(1*H*)-one (156 mg, 2.12 mmol) and the compound of formula 22 (diastereomer a, 96 mg, 0.15 mmol) are dissolved in CH₂Cl₂ (5 ml) at 25°C. After stirring for 1 h the reaction mixture is poured in a mixture of a sat. aq. NaHCO₃ soln. (10 ml) and a sat. aq. Na₂S₂O₃ son. (10 ml), extracted three times with tert-butyl methyl ether (20 ml each), dried over MgSO₄, and evaporated *in vacuo*. Flash chromatography (acetone/pentane 1:3) gives 75 mg (78 %) of compound (23) as a colorless foam. 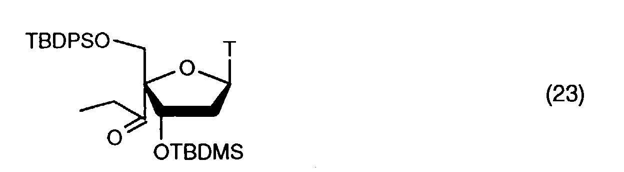
IR (KBr): 3414, 3192, 3072, 2955, 2931, 2886, 2858, 1718, 1700, 1472, 1428, 1279, 1254, 1114, 1076, 1035, 958, 834, 779, 703, 505.
¹H-NMR (CDCl₃): -0.03 (*s*, CH₃―Si), 0.03 (*s*, CH₃―Si), 0.84 (*s*, *t*-Bu―Si), 0.96 (*t*, *J*=7.1, CH₂― C*H*₃), 1.10 (*s*, *t*-Bu―Si), 1.59 (*s*, CH₃―C(5)), 2.28 (*m*, H―C(2')), 2.58 (*qd*, *J*=7.1, *J*=19.4, CH₂(a)―C(O)), 2.82 (*qd*, *J*=7.1, *J*=19.4, CH₂(b)―C(O)), 3.94 (*d*, *J*=11.2, H―C(5'a)), 4.11 (*d*, *J*=11.2, H―C(5'b)), 4.49 (*d*, *J*=3.6, H―C(3')), 6.65 (*dd*, *J*=5.7, *J*=8.9, H―C(1')), 7.42 (*m*, Hₐᵣₒₘ), 7.63 (*m*, H―C(6), Hₐᵣₒₘ), 9.05 (*s*, NH).
¹³C-NMR (CDCl₃): -5.3 (CH₃―Si), -5.2 (CH₃―Si), 6.6 (*C*H₃―CH₂), 12.0 (*C*H₃―C(5)), 17.9 (Me₃*C*―Si), 19.4 (Me₃*C*―Si), 25.6 ((*C*H₃)₃―C-Si), 27.0 ((*C*H₃)₃―C-Si), 33.6 (*C*H₂―C(O)), 41.6 (C(2')), 67.0 (C(5')), 76.0 (C(3')), 86.2 (C(1')), 96.5 (C(4')), 111.3 (C(5)), 128.0, 128.1 (m-Cₐᵣₒₘ), 130.1, 130.3 (p-Cₐᵣₒₘ), 132.0, 132.6 (i-Cₐᵣₒₘ), 135.26 (C(6)), 135.31, 135.5 (o-Cₐᵣₒₘ), 150.3 (C(2)), 163.8 (C(4)), 210.8 (Et-*C*(O)).
FAB-MS: 651 (3, [M+1]⁺). Anal. calc. for C₃₅H₅₀N₂O₆Si₂ (650.97):
   C 64.58, H 7.74, N 4.30; found: C 64.46, H 7.61, N 4.11.

### Example 20: 4'-Propionyl-2'-deoxy-thymidine

To a solution of compound 23 (0.23 g, 0.35 mmol) in THF (15 ml) a 1M solution of tetrabutylammonium fluoride (TBAF) (0.9 ml, 0.9 mmol) is added at 25°C and stirred for 5 h. Then, silica gel (5 g) is added to the reaction mixture and the solvent is evaporated *in vacuo*. Flash chromatography (ethyl acetate/acetonitrile 4:1), removing of the solvent in vacuo, dissolving in water and lyophylization gives 72 mg (67%) of compound of formula 24 as a colorless foam. 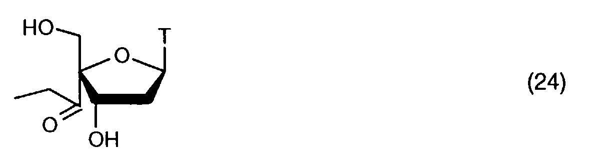
IR (KBr): 3412, 3063, 2979, 2939, 1700, 1474, 1406, 1376, 1115, 1047, 962, 779, 572.
¹H-NMR (CD₃OD): 0.99 (*t*, *J*=7.2, CH₂―CH₃), 1.89 (*d*, *J*=1.2, CH₃―C(5)), 2.30 (*m*, H―C(2')), 2.68 (*m*, CH₂―C(O)), 3.84 (*d*, *J*=11.7 H―C(5'a)), 3.89 (*d*, *J*=11.7 H―C(5'b)), 4.46 (*m*, H― C(3')), 6.57 (*dd*, *J*=6.1, *J*=8.5, H―C(1')), 7.85 (*d*, *J*=1.2, H―C(6)).
¹³C-NMR (CD₃OD): 7.3 (CH₂―*C*H₃), 12.5 (*C*H₃―C(5)), 34.8 (*C*H₂―(O)), 41.2 (C(2')), 65.6 (C(5')), 75.5 (C(3')), 87.8 (C(1')), 98.0 (C(4')), 112.0 (C(5)), 138.2 (C(6)), 152.5 (C(2)), 166.6 (C(4)), 214.3 (CH₃―*C*(O)).
FAB-MS: 299 (31, [M+1]⁺). Anal. calc. for C₁₃H₁₈N₂O₆•0.35 H₂0 (304.61):
   C 51.26, H 6.19, N 9.20, O 33.35; found: C 51.13, H 6.24, N 9.05, O 33.05.

### Example 21: Use of modified nucleotide in chain elongation

The triposphates of the modified nucleotides are synthesized as described by Kovács & Ötvös (Tetrahedron Lett. (1988), **29**, 4525-2528)
5' P³²-GTGGTGCGAATTCTGTGGAT-OH SEQ ID NO:1
3' HO-CACCACGCTTAAGACACCTA**GTCGTTCCTACTTGCTGGCT**-OH SEQ ID NP:2

A solution of template (3.0 pmol of SEQ ID NO:2) and radioactive primer (0.8 pmol of SEQ ID NO:1) in 72 µl buffer (20 mM Tris-HCl, pH 7.5; 6 mM MgCl₂; 40 mM KCl; 0.5 mM DTT) are heated to 80°C and allowed to cool down to 25°C within one hour. To this solution polymerase (see table 1) is added. Aliquots of thereof are mixed with 4 µl of a solution comprising dATP, dGTP, dCTP and the modified nucleotide (see table 1), resulting in a final concentration of dATP, dGTP and dCTP as provided in table 1 and a final concentration of the modified nucleotide of 100 µM. The solutions are incubated at 37°C and the elongation reaction is stopped by addition of 5 µl of a solution of formamide comprising 5 mM EDTA (pH 8.0).

**Table 1**

| Enzyme | Amount of enzyme | Buffer | Concentration of dATP, dGTP, dCTP |
|---|---|---|---|
| Klenow | 1.5 units | 10 mM Tris-HCl (pH 7.5), 5 mM MgCl₂, 7.5 mM DTT | 0.5 µM each |
| T7 sequenase version 2 | 0.13 units | 40 mM Tris-HCl (pH 7.5), 20 mM MgCl₂, 50 mM NaCl | 2.5 µM each |
| AMV RT | 2.0 units | 50 mM Tris-HCl (pH 8.3), 8 mM MgCl₂, 30 mM KCl | 0.5 µM each |
| M-MuLV RT | 9 units | 50 mM Tris-HCl (pH 8.3), 8 mM MgCl₂, 10 mM DTT | 15.0 µM each |
| HIV-1RT | 0.13 units | 20 mM Tris-HCl (pH 7.5), 6 mM MgCl₂, 40 mM KCl, 0.5 mM DTT | 1.0 µM each |

3-5 µl of these solutions are subjected to a polyacrylamide gel (19 % acrylamide, 7 M urea). After electrophoresis the gel is transferred to a filter paper, dried and analyzed using autoradiography (see table 2).

**Table 2**

| 25, modified nucleotide | Klenow | T7 sequenase version 2 | AMV RT | M-MuLV RT | HIV-1RT |
|---|---|---|---|---|---|
| R¹=CH₃ | ++ | ++ | ++ | ++ | ++ and elongation |
| R¹=CH₃CH₂ | + | ++ | ++ | ++ | ++ |
| R¹=C₆H₅ | - | - | ++ | - | ++ |
| R¹=t-butyl | - | - | - | - | - |
| + = slow addition of modified nucleotide to the primer ++ = good addition of modified nucleotide to the primer - = no addition of modified nucleotide to the primer | | | | | |

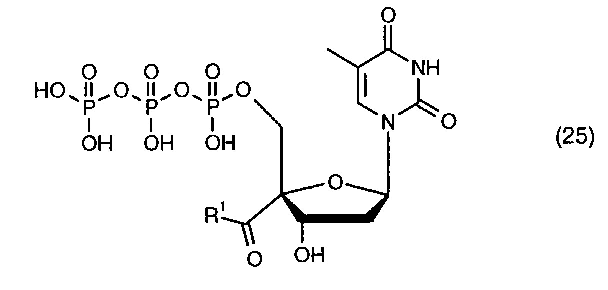

### Example 22: Photolysis of the modified oligonucleotides

A solution of template (3.0 pmol of SEQ ID NO:2) and radioactive primer (0.8 pmol of SEQ ID NO:1) in 72 µl buffer (20 mM Tris-HCl, pH 7.5; 6 mM MgCl₂; 40 mM KCl; 0.5 mM DTT) are heated to 80°C and allowed to cool down to 25°C within one hour. To this solution 2.4 units of HIV-1 RT (24 µl of a 0.1 units/µl solution in 20 mM Tris-HCl (pH 7.5), 1 mM EDTA (pH 8.0), 1 mM DTT, 15 % Glycerol, 0,2 % Bovine serum albumin) is added. The solution is added to 64µl of a solution comprising dATP, dGTP, dCTP and the modified nucleotide 25 wherein R¹ is CH₃, resulting in a final concentration of dATP, dGTP and dCTP of 1.25 µl each and a final concentration of the modified nucleotide of 100 µM. After 4 h 160 µl of a 5 M NH₄OCOCH₃ solution and 1000 µl ethanol are added an cooled at -20°C for 1 h. The solution is centrifuged at 20800g, the supernatant is decanted and the residue resolved in 200 µl buffer (100 mM NaCl, 1 mM phosphate-buffer (pH 7.0), 0.01 mM EDTA).

The solution is irradiated at 15°C (Osram Hochdrucklampe, 500 W, 295 nm filter). After irradiation for 40 min. 20 µl 3M NaOCOCH₃ and 800 µl ethanol are added at - 20°C for 1h. The solution is centrifuged at 20800 g, the supernatant decanted and the residue resolved in 100 µl buffer (10mM Tris-HCl, pH 8.3). A 5 gl aliquot of this solution is mixed with 5 µl of a solution of formamide comprising 5 mM EDTA (pH 8.0) and loaded on a gel as described above. The autoradiogram shows the complete cleavage of the enzymatically synthesized oligonucleotides.

## Claims

1. A compound of formula 1 or 2 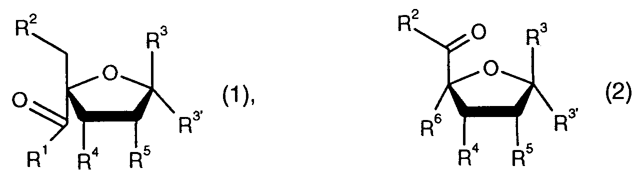 wherein
R¹ is CH₃, C₂H₅, CH₂X, CHX₂, CX₃, C(O)H, C(O)C₁-C₄alkyl, CH₂OH, CH₂-O-C₁-C₄alkyl, CH₂-O-phenyl, phenyl, or phenyl substituted with nitro or X;
R² is OR⁷, mono-, di-, or triphosphate, or esters or prodrugs thereof;
one of the residues R³ and R³ 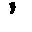 is a purine or pyrimidine residue or an analogue thereof, and the other is hydrogen;
R⁴ and R⁵ are independent of one another H, OR⁷, O-C₁-C₄alkylNHR⁷, O-C₁-C₄alkylNR⁷₂, -O-(CH₂-CH₂-O)₁₋₄R⁷ or -O-CH₂-C(OR⁷)H-CH₂-OR⁷;
R⁶ is H, OH, CH₂OH, CH₃, CH₂CH₃, CH₂CH₂OH;
R⁷ is H or an NH or OH-protecting group;
R⁸ is H or an OH-protecting group;
X is F, Cl, Br or I;
or a pharmaceutically acceptable salt or prodrug thereof.

2. A compound according to claim 1, wherein R¹ is CH₃, C₂H₅, CF₃, CH₃OH, CH₃-O-CH₃, phenyl and phenyl substituted with nitro.

3. A compound according to claim 1, wherein R¹ is CH₃, C₂H₅ phenyl.

4. A compound according to claim 1, wherein R¹ is CH₃.

5. A compound according to claim 1, wherein R² is mono-, di-, or triphosphate; or HO.

6. A compound according to claim 1, wherein one of the residues R³ and R³ 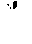 is adenine, inosine, N-methyladenine, N-benzoyladenine, 2-methylthioadenine, 2-aminoadenine, 6-hydroxypurine, 2-amino-6-chloro purine, 2-amino-6-methylthiopurine, guanine, N-isobutyrylguanine, uracil, thymine, cytosine, 5-fluorouracil, 5-chlorouracil, 5-bromouracil, dihydrouracil and 5-methylcytosine; or their base-protected derivatives; and the other residue is hydrogen.

7. A compound according to claim 1, wherein one of the residues R³ and R³ 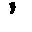 is adenine, inosine, guanine, uracil, thymine or cytosine; and the other residue is hydrogen.

8. A compound according to claim 1, wherein one of the residues R³ and R³ 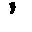 is thymine; and the other residue is hydrogen.

9. A compound according to claim 1, wherein R⁴ and R⁵ are independent of one another H, or OR⁷.

10. A compound according to claim 1, wherein R⁴ is OH.

11. A compound according to claim 1, wherein R⁵ is H.

12. A compound according to claim 1, wherein R⁶ is H, OH or CH₂OH.

13. A compound according to claim 1, wherein R⁷ is H or C₁-C₄alkyl.

14. A compound according to claim 1, wherein R⁷ is H.

15. A compound according to claim 1, wherein X is F or Cl.

16. A compound according to claim 1, wherein X is F.

17. A compound according to claim 1, which has formula 1.

18. A compound according to claim 1, wherein R³ 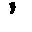 is hydrogen

19. Use of a compound according to claim 1 in a method of treatment.

20. Use of a compound according to claim 1 in a method of treatment, wherein the active compound according to claim 1 is formed *in vivo*.

21. Use of a compound according to claim 1 in a method of treatment of retro virus induced diseases.

22. Use of a compound according to claim 1 in a method of treating AIDS.

23. Use of a compound according to claim 1 in a method of inhibiting the proliferation of retro viruses.

24. Use of a compound according to claim 1 in a method of inhibiting the proliferation of MMTV, MLV, spumavirus, HTLV, BLV, lentivirus, HIV, HSV and SIV.

25. Use of a compound according to claim 1 in a method of inhibiting HIV.

26. Pharmaceutical composition comprising a compound according to claim 1 optionally together with pharmaceutical acceptable carrier.

27. Method for the preparation of a pharmaceutical composition according to claim 26.

28. Use of the compound according to claim 1 for the inhibition of reverse transcriptase.

29. Use of a compound according to claim 1 in a screen.

30. Use of a compound according to claim 29 in a screen for the identification of the presence or absence of reverse transcriptase.

31. Use of a compound according to claim 29 in a screen for the identification of HIV-1RT.

32. Use of a compound according to claim 1 for the generation of photolytically cleavable DNA and RNA and analogues thereof.

33. A photocleavable linker comprising a compound according to claim 1.

34. Method for the synthesis of a compound according to claim 1.
